# EUROPEAN PATENT APPLICATION

(11) **EP 4 235 480 A1**
(43) Date of publication of application: **30.08.2023**
(21) Application number: 23158075.4
(22) Date of filing: 22.02.2023
(51) Int. Cl.: G06F 30/00, G06F 30/12, A61B 34/10, G06F 113/10, G06F 111/16

(54) **VOLUME SPLITTING COMPLEX SHAPES**

(30) Priority: 23.02.2022 US 202263313170 P
(71) Applicant: Biomet 3I, LLC, Florida 33410 (US)
(72) Inventor: MOTRONI, Alessandro, Palm Beach Gardens, 33410 (US); TOCCHETTI, Angelo, Palm Beach Gardens, 33410 (US)
(74) Representative: Platzöder, Michael Christian

(57) **Abstract**

A computing device (200) is provided that includes a processor (204) and memory (216) including data that, when processed by the processor (204), enables the computing device (200) to: import a freeform object into a Computer-Aided Drafting (CAD) environment (228); facilitate a customization of the freeform object within the CAD environment by independent manipulation of one or more control points in at least two dimensions, where the one or more control points are defined at a surface of the freeform object and, when manipulated, result in a changed configuration of the freeform object in the at least two dimensions; determine an overlapping volume in the CAD environment (228) that is shared between the freeform object and another object; and perform one or more Boolean operations to convert the overlapping volume into a split volume.

## Description

### CROSS-REFERENCE TO RELATED APPLICATIONS

This application claims the benefit of U.S. Provisional Application No. 63/313,170, filed on February 23, 2022, which is incorporated herein by reference in its entirety.

### BACKGROUND

The present disclosure is generally directed to Computer-Aided Drafting (CAD), and relates more particularly to CAD tools used to support surgical procedures, such as dental procedures.

The use of surgical navigation in the head and neck has made significant advancements recently. Presently, systems are used in otolaryngology, neurosurgery, and oral and maxillofacial surgery for a variety of surgical procedures.

Recent advances allow dentists, oral and maxillofacial surgeons, and maxillofacial prosthodontists to place dental implants accurately or plan surgeries based on a patient's actual anatomy. Some CAD tools even support the creation of objects within the CAD environment based on images taken of a patient's anatomy. Most CAD tools, however, are limited in the types of objects or primitives they can create. Furthermore, existing CAD tools limit the manner in which objects or primitives can be manipulated, especially when dealing with overlapping volumes or complex volumes, which makes customized surgical planning more difficult. These limitations of existing CAD tools can lead to less-than-optimal surgical results and/or extended patient time in the chair during surgery.

### BRIEF SUMMARY

Embodiments of the present disclosure were contemplated to address the above-noted issues of existing CAD tools and surgical procedures supported by inflexible CAD tools. Specifically, but without limitation, embodiments of the present disclosure propose an improved and highly-flexible CAD tool that supports the creation of freeform objects within a CAD environment. The freeform objects can be merged with other objects in the CAD environment and/or used as a cut-type object to remove certain portions of objects within the CAD environment. More specifically, freeform objects can be used to realize split volumes with complex shapes using one or more Boolean operations.

One aspect of the present disclosure provides a computing device having a processor and memory. The memory may include data and/or instructions that, when processed by the processor, enable the computing device to:
import a freeform object into a Computer-Aided Drafting (CAD) environment;
facilitate a customization of the freeform object within the CAD environment by independent manipulation of one or more control points in at least two dimensions, wherein the one or more control points are defined at a surface of the freeform object and, when manipulated, result in a changed configuration of the freeform object in the at least two dimensions;
determine an overlapping volume in the CAD environment that is shared between the freeform object and another object; and
perform one or more Boolean operations to convert the overlapping volume into a split volume.

Another aspect of the present disclosure provides a surgical system that includes:
a computing device configured to import an object into a Computer-Aided Drafting (CAD) environment, enable creation of one or more control points on a surface of the object, and further enable customization of the object within the CAD environment via independent manipulation of the one or more control points in two or more dimensions even when the object overlaps at least one other object in the CAD environment.

Another aspect of the present disclosure provides a method of creating and/or manipulating objects in a CAD environment, the method including:
importing a first object into a Computer-Aided Drafting (CAD) environment;
importing a second object into the CAD environment;
enabling independent manipulation of the first object and/or second object relative to one another within the CAD environment even when the first object overlaps the second object;
determining an overlapping volume in the CAD environment that is shared between the first object and the second object;
creating a third volume based on performing one or more Boolean operations to convert the overlapping volume into the third volume; and
presenting the third volume within the CAD environment as a portion of the first object and/or second object.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2 is a block diagram illustrating details of a computing device according to at least one embodiment of the present disclosure;
Figs. 3A - 3T illustrate a number of Graphical User Interface (GUI) views presented by a CAD tool during one or more workflows according to at least one embodiment of the present disclosure;
Figs. 4A - 4J illustrate a number of GUI views presented by a CAD tool during one or more additional workflows according to at least one embodiment of the present disclosure;
Figs. 5A - 5E illustrate a number of GUI views presented by a CAD tool during one or more additional workflows according to at least one embodiment of the present disclosure;
Fig. 6 is a flow diagram illustrating a first method according to at least one embodiment of the present disclosure;
Fig. 7 is a flow diagram illustrating a second method according to at least one embodiment of the present disclosure; and
Fig. 8 is a flow diagram illustrating a third method according to at least one embodiment of the present disclosure.

### DETAILED DESCRIPTION

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to the operator or user of the system, and further from the region of surgical interest in or on the patient, and distal being closer to the region of surgical interest in or on the patient, and further from the operator or user of the system.

Embodiments of the present disclosure provide solutions to a number of different problems associated with CAD tools and the use of CAD tools in connection with planning or performing surgical procedures. As an example, embodiments of the present disclosure provide CAD tools that are configured to generate and manipulate freeform objects across multiple dimensions. Embodiments of the present disclosure also provide CAD tools that enable volume splitting of complex shapes, which may or may not include the use of freeform objects created by a user. Embodiments of the present disclosure also provide CAD tools that can communicate with a manufacturing system, which can produce complex physical items according to the objects defined in the CAD tools. The flexibility and customization offered by the CAD tools proposed herein supports the ability to rapidly create customized surgical guides, stacked guides, stacked components, cutting templates, or the like in real-time, which translates to improved patient outcomes and/or reduced chair time for a patient during a surgical procedure.

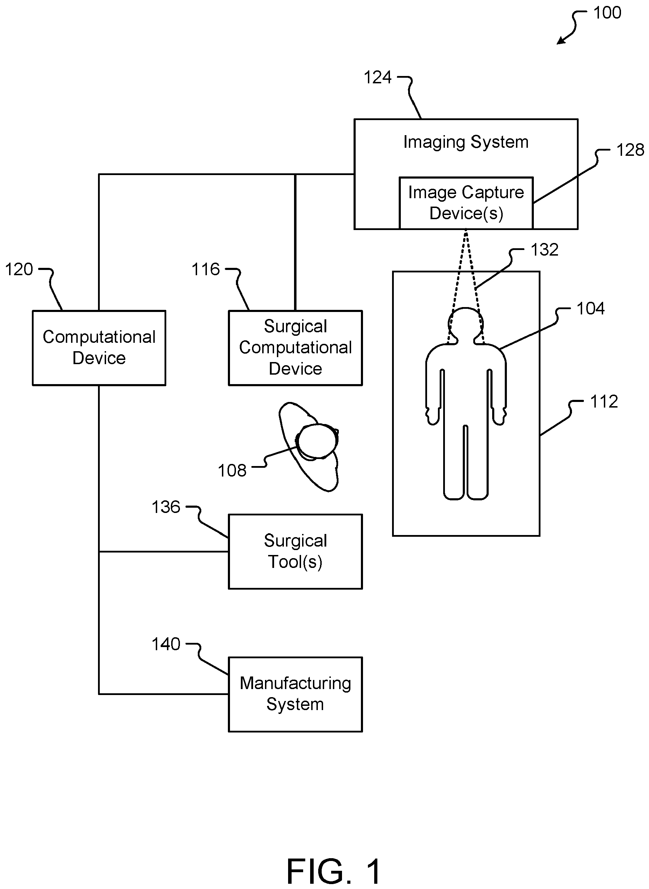

Turning initially to Fig. 1, a system 100 is illustrated in accordance with at least some embodiments of the present disclosure. The system 100 may correspond to a surgical system, computing system, communication system, or combination thereof. In some embodiments, the system 100 includes a surgical system that enables a care provider 108 to perform a surgical procedure on a patient 104. Illustratively, the system 100 may include a table or chair 112 that supports the patient 104 during the surgical procedure. The system 100 is also shown to include one or more computational devices 116, 120, an imaging system 124, surgical tool(s) 136, and a manufacturing system 140.

The computational devices 116, 120 may include one or more computational devices 116 that are accessible to the care provider 108 during the surgical procedure 116. The computational devices 116, 120 may also include one or more computational devices 120 that are remotely located with respect to the table or chair 112. Either computational device 116, 120 may be configured to provide CAD tools to the care provider 108 and may facilitate the planning of a surgical procedure. In some embodiments, the computational devices 116, 120 may enable the care provider 108 to plan a surgical procedure before the patient 104 is on the table or chair 112. Alternatively or additionally, the computational devices 116, 120 may enable the care provider 108 to update plans for a surgical procedure during the surgical procedure (e.g., while the patient 104 is on the table or chair 112). As an example, CAD tools and/or imaging tools provided by the computational devices 116, 120 may enable the care provider 108 to plan surgical maneuvers (e.g., plan a cut/incision, plan an implant placement, check an implant placement, visualize an implant placement, adjust an implant placement, plan a bone graft, etc.), build surgical tools (e.g., build a surgical guide, build a collection of surgical guides, customize implants, customize implant-placement tools, etc.), and perform other tasks during a surgical procedure.

Although the surgical tool(s) 136 and manufacturing system 140 are only depicted as being connected to the computational device 120 for ease of illustration, it should be appreciated that the surgical computational device 116 may also be connected to the surgical tool(s) 136 and/or manufacturing system 140. While two separate computational devices 116, 120 are illustrated, it should be appreciated that the system 100 may include a single computational device that is useable before a surgical procedure and during the surgical procedure. In other words, two or more computational devices are not a requirement of the system 100, but rather are illustrated to show that different computational devices may be used by the care provider 108 during different phases of planning a surgical procedure.

The imaging system 124 may include one or more image capture device 128 that are configured to capture images of the patient 104 or patient anatomy. In some embodiments, the image capture devices 128 are configured to capture image(s) of patient anatomy within a field of view 132. As an example, the image capture device(s) 128 may be operable to image anatomical feature(s) (e.g., a bone, veins, soft tissue, etc.) and/or other aspects of patient anatomy to yield image data (e.g., image data depicting or corresponding to a bone, veins, soft tissue, etc.). "Image data" as used herein refers to the data generated or captured by an image capture device 128, including in a machine-readable form, a graphical/visual form, and in any other form. In various examples, the image data may comprise data corresponding to an anatomical feature of a patient, or to a portion thereof. The image data may be or comprise a preoperative image, an intraoperative image, a postoperative image, or an image taken independently of any surgical procedure.

In some embodiments, an image capture device 132 may be capable of taking a 2D image or a 3D image to yield the image data. The image capture device 132 may be or comprise, for example, any device utilizing X-ray-based imaging (e.g., a fluoroscope, a CT scanner, or other X-ray machine), a Magnetic Resonance Imaging (MRI) scanner, an ultrasound imaging device, an optical coherence tomography (OCT) scanner, an endoscope, a microscope, an optical camera, a thermographic camera (e.g., an infrared camera), a radar or LIDAR system (which may comprise, for example, a transmitter, a receiver, a processor, and one or more antennae), or the like. The image capture device 132 may be contained entirely within a single housing, or may comprise a transmitter/emitter and a receiver/detector that are in separate housings or are otherwise physically separated.

In some embodiments, the imaging system 124 may include more than one image capture device 132. The imaging system 124 is capable of obtaining image(s) of patient anatomy in the form of image data, then providing the image(s) to the computational device(s) 116, 120, where one or more objects or models of patient anatomy may be uploaded into a CAD environment. For instance, images captured with the imaging system 124 may be used to generate one or more models of patient anatomy that can then be visualized and manipulated within a CAD tool executed on the computational device 116, 120. As a more specific, but non-limiting example, the CAD tools executed on the computational device 116, 120 may be configured to generate and manipulate a model of patient anatomy (e.g., hard tissue, soft tissue, combinations of hard/soft tissue, etc.) as will be described in further detail herein.

The surgical tool(s) 136 may include any number of known or yet-to-be developed surgical instruments. In some embodiments, the surgical tool(s) 136 may include handheld tools that are useable by the care provider 108 during the surgical procedure. Examples of such tools include, without limitation, drills, scissors, blades, knives, scalpels, retractors, implants, portions of implants, implant kits, surgical guides, screws, sutures, etc. The surgical tool(s) 136 may alternatively or additionally include robotic surgical tools or robotic surgical systems. For instance, the surgical tool(s) 136 may include a surgical robot that helps the care provider 108 perform a surgical procedure either semi-autonomously or fully-autonomously.

The manufacturing system 140 may be configured to create, in real-time (e.g., during a surgical procedure), surgical tool(s) 136. For example, the manufacturing system 140 may interact with CAD tools provided by the computational device 116, 120 to create surgical implants, surgical guides, stacked surgical guides, or the like. The manufacturing system 140 may be co-located with the table or chair 112 or may be located remotely from the table or chair 112. As a non-limiting example, the manufacturing system 140 may include a three-dimensional (3D) printer, a Computer Numerical Control (NCN) machine, a turning machine, a shaper/planer, a drilling machine, a milling machine, a grinding machine, a power saw, a press, a lamination machine, or a combination thereof. The manufacturing system 140 may be configured to receive one or more electronic files from a computational device 116, 120 and manufacture an object in accordance with dimensions of the object defined within the electronic file. As a non-limiting example, the manufacturing system 140 may include a 3D printer that is configured to produce a surgical guide or collection of surgical guides during a surgical procedure in accordance with object data received from a computational device 116, 120. The surgical guide or collection of surgical guides may be produced during a surgical procedure based on images captured of the patient 104 while on the table or chair 112 (e.g., while sedated for the surgical procedure).

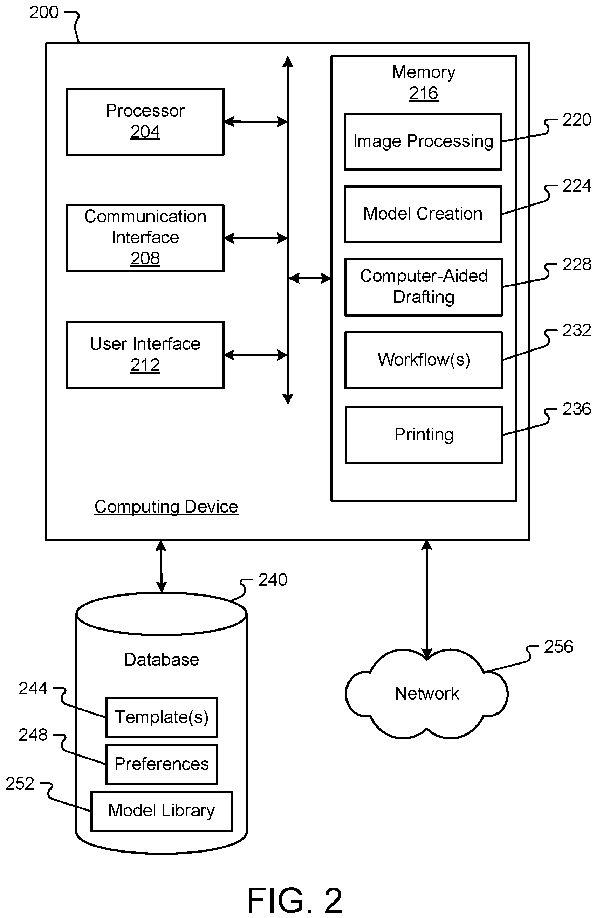

With reference now to Fig. 2, additional details of a computing device 200 will be described in accordance with at least some embodiments of the present disclosure. The computing device 200 may correspond to an example of the computational device 116 and/or computational device 120. The computing device 200 is shown to include a processor 204, a communication interface 208, a user interface 212, and memory 216. The computing device 200, in some embodiments, is interconnected with a database 240 and a broader communication network 256.

The processor 204 of the computing device 200 may be any processor described herein or any similar processor. The processor 204 may be configured to execute instructions or machine learning models (e.g., neural networks, decision trees, etc.) stored in the memory 216 (e.g., data), which may cause the processor 204 to carry out one or more computing steps. Illustratively and without limitation, the processor 204 may be configured to provide CAD tools to a user of the computing device 200 when executing contents of the memory 216.

The memory 216 may be or comprise RAM, DRAM, SDRAM, other solid-state memory, any memory described herein, or any other tangible, non-transitory memory for storing computer-readable data and/or instructions. The memory 216 may store information or data useful for completing, for example, any step of the methods or workflows described herein. The memory 216 may store, for example, one or more image processing algorithms 220, model creation functions 224, CAD tools 228, CAD workflows 232, and/or printing functions 236. Such instructions, data, or algorithms may, in some embodiments, be organized into one or more applications, modules, packages, layers, or engines. The algorithms, data, and/or instructions may cause the processor 204 to manipulate data stored in the memory 216 and/or received from or via the imaging system 124, the database 240, and/or the communication network 256.

The image processing 220, when executed by the processor 204, may enable the computing device 200 to cooperate with the imaging system 124 to obtain and use images of the patient 104 or patient anatomy. In some embodiments, the image processing 220 may be configured to receive patient images (e.g., preoperative patient images, intraoperative patient images, and/or postoperative patient images), receive object images, etc., and prepare the images for processing by other components of the computing device 200. Illustratively, the image processing 220 may be configured to receive image data and format the image data for consumption by the model creation 224 and/or CAD tools 228. In some embodiments, the image processing 220 may be configured to transform an image or image data into a different consumable format by converting the image from one digital format to another digital format, by performing pixel analysis to determine locations or boundaries of objects, by identifying locations of fiducials in an image, by compressing an image, by decompressing an image, by overlaying object models on an image, and/or any other task associated with preparing an image for consumption by the model creation 224 for use by the CAD tools 228.

The model creation 224 is shown as being separate from the CAD tools 228, but may be incorporated into the CAD tools 228. In some embodiments, the model creation 224 is configured to convert image data from the image processing 220 into one or more models of an anatomical element. For instance, the model creation 224 may be configured to generate an object within the CAD tools that represents the physical dimensions of a patient's 104 anatomical part. As a more specific but non-limiting example, the model creation 224 may be configured to generate an electronic file representing a patient's anatomy (e.g., gingiva, teeth, mandibular jawbone, elements of the mandible, maxillary bone, elements of the maxilla, nerves, soft tissue, etc.). The objects or models created by the model creation 224 may be viewable and capable of manipulation with the CAD tools 228.

To create a prosthesis, the dental region of the patient 104 may be placed within the field of view 132 and be is scanned, as described above. Alternatively or additionally, the model creation 224 may use other inputs to generate an electronic file representing the patient's 104 anatomy. For instance, the model creation 224 may be configured to generate models using one or more of a stone model made from impression material, laser scanning techniques, a photographic scanning technique, or a mechanical sensing technique. Alternatively or additionally, the care provider 108 can photograph implants and other components that have been placed into or adjacent the patient's 104 mouth. The model creation 224 may also be configured to accurately relate the gingival margin for all mold, model, implant and abutment dimensions.

The CAD tools 228 may be configured to allow visualization and manipulation of objects within a CAD environment. For instance, the CAD tools 228 may enable a visualization of patient 104 anatomy as well as possible implants to be placed within the patient 104 anatomy. Additional capabilities and features of the CAD tools 228 will be described in further detail herein.

The workflow(s) 232 may represent various guides for steps to be taken with the CAD tools 228. Although depicted as separate from the CAD tools 228, it should be appreciated that the workflow(s) 232 may be provided as part of the CAD tools 228. In some embodiments, the workflow(s) 232 represent an ordered series of steps to take within the CAD environment as the care provider 108 manipulates the objects with the CAD tools 228. Workflow(s) 232 may include prosthesis placement/planning, surgical guide creation, bone graft planning, incision planning, drill planning, etc.

The printing 236 may correspond to an Application Programming Interface (API) that facilitates communication between the CAD tools 228 and the manufacturing system 140. In some embodiments, the printing 236 functionality enables one or more objects in the CAD environment to be produced via the manufacturing system 140. As a more specific example, the printing 235 functionality may generate one or more electronic files that describe physical dimensions of an object (or multiple objects) in the CAD environment along with instructions for the manufacturing system 140 to accurately produce/replicate the object using an appropriate manufacturing technique (e.g., 3D printing).

In some embodiments, the CAD tools 228 may operate with assistance of data from the database 240. Examples of data that may be stored in database 240 and may be used by the CAD tools 228 include template(s) 244, preferences 248, and a model library 252. While illustrated as being in a separate database 240, it should be appreciated that some or all of the elements shown in the database 240 could be included in memory 216 without departing from the scope of the present disclosure.

The template(s) 244 and/or model library 252 may include one or more electronic files describing objects for use by the CAD tools 228. In some embodiments, objects may be created or manipulated within the CAD environment, then the objects may be saved as templates 244 for later use in a different surgical procedure. For instance, a care provider 108 may create one or more templates that can be slightly adjusted or customized for use with different patients. Objects can be loaded into the CAD environment by the CAD tools 228 using the templates 224. Similarly, presentation and application preferences for the CAD tools 228 may be maintained and referenced as part of the preferences 248. The preferences 248 may correspond to one or more values or settings used at an application level by the CAD tools 228. The preferences 248 may be changed, saved, or reset by the care provider 108.

The model library 252 may include one or more templates 244. In some embodiments, however, the model library 252 may store electronic files for models or objects to be used during a surgical procedure. As an example, the model library 252 may include files describing physical dimensions of implants or implant kit components. The dimensions of the objects stored in the model library 252 may be defined by the manufacturer of the implants. Objects loaded from the model library 252 may or may not be adjustable or have control points that facilitate an adjustment of relative dimensions by the CAD tools 228 whereas objects loaded from the template(s) 244 may be adjustable or have control points that facilitate an adjustment of relative dimensions by the CAD tools 228.

The communication interface 208 may be used for receiving image data or other information from an external source (such as the imaging system 124, the database 240, the network 256, and/or any other system or component not part of the system 100). The communication interface 208 may provide connectivity between the computing device 200 and the communication network 256, thereby enabling machine-to-machine communication with other devices of the system 100. The communication interface 208 may comprise one or more wired interfaces (e.g., a USB port, an ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 208 may be useful for enabling the computing device 200 to communicate with one or more other processors or computing devices 200, whether to reduce the time needed to accomplish a computing-intensive task or for any other reason.

The user interface 212 may be or comprise a keyboard, mouse, trackball, monitor, television, screen, touchscreen, and/or any other device for receiving information from a user and/or for providing information to a user. The user interface 212 may be used, for example, to receive a user selection or other user input regarding any step of any method described herein. Although the user interface 212 is shown as part of the computing device 200, in some embodiments, the computing device 200 may utilize a user interface 212 that is housed separately from one or more remaining components of the computing device 200. In some embodiments, the user interface 212 may be located proximate one or more other components of the computing device 200, while in other embodiments, the user interface 212 may be located remotely from one or more other components of the computing device 200. Illustratively, the user interface 212 may include a user input device, a user output device, and a combination user input/user output device (e.g., a touch-sensitive display device).

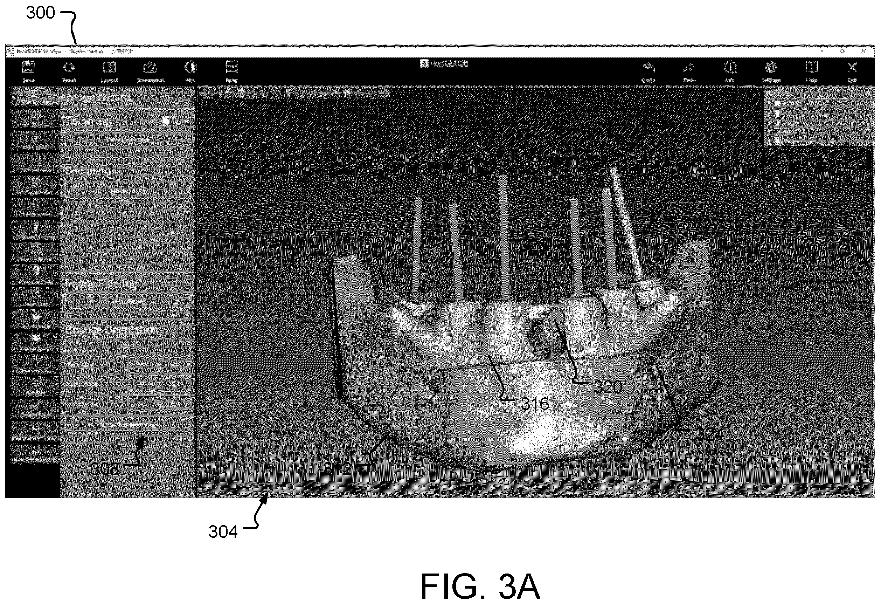

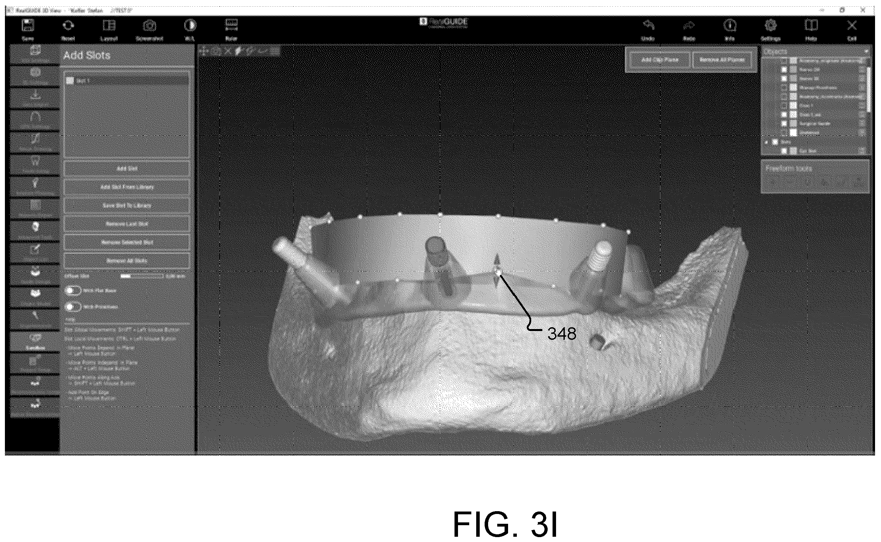

Referring now to Figs. 3A - 5G, various workflows and capabilities of the CAD tools 228 will be described in accordance with at least some embodiments of the present disclosure. The workflows and capabilities are shown and described in a particular order, but it should be appreciated that the CAD tools 228 can be configured to enable the order of operations in any particular workflow to be changed, skipped, modified, or the like. More specifically, the CAD tools 228 are flexibly configured to allow the care provider 108 to take any step or combination of steps in any order desired. The illustrated steps are shown for purposes of illustration and should not be construed as limiting embodiments of the present disclosure.

Referring initially to Figs. 3A - 3T, a first workflow or set of workflows will be described in accordance with at least some embodiments of the present disclosure. Fig. 3A illustrates a first view of a GUI 300 provided by the CAD tools 228. The GUI 300 may be presented via a user interface 212. The GUI 300 is shown to include a presentation frame or application window that provides one or more icons, visualization tools, and the like. The GUI 300 illustrated in Fig. 3A is shown to include a CAD environment 304 in which one or more objects are viewable, manipulatable, and/or rotatable. The GUI 300 also includes an image wizard toolbar 308. The image wizard toolbar 308 may be include tools that can be selected to change an orientation or presentation of objects within the CAD environment 304. The one or more objects shown in the CAD environment 304 include a first object 312, a second object 316, a third object 320, a fourth object 324, and a fifth object 328. It should be appreciated that a greater or lesser number of objects can be presented simultaneously such that relative dimensions of various objects can be visualized for purposes of planning a surgical procedure. As shown, one or more objects (e.g., the first object 312 and fourth object 324) may correspond to objects representing patient 104 anatomical features whereas other objects (e.g., second object 316, third object 320, and fifth object 328) may correspond to man-made objects. The objects representing patient 104 anatomical features may include hard tissue objects (e.g., bones, teeth, etc.) or soft tissue objects (e.g., nerves, arteries, gingiva, tongue, etc.). The objects representing man-made objects may include implants, screws, surgical guides, stacked surgical guides, alignment mechanisms, fasteners, or the like.

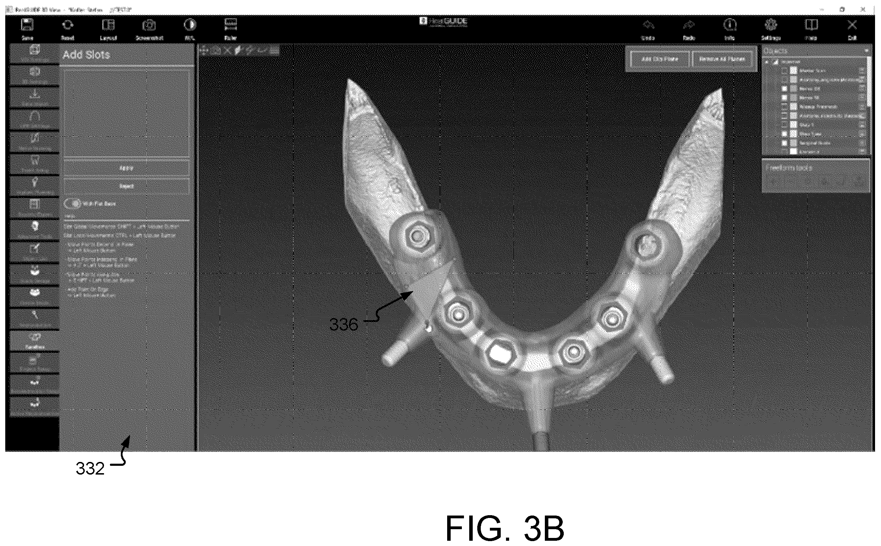

Fig. 3B illustrates a view of the GUI 300 in which the objects are viewed from a top view. Fig. 3B also illustrates a slot creation toolbar 332. As will be described in further detail herein a slot may correspond to a particular type of object within the CAD environment 304. In some embodiments, a slot may refer to a cut-type of object 336 that can be created and manipulated within the CAD environment 304. The slot creation toolbar 332 may present features and functions that facilitate a care provider's 108 creation and manipulation of a cut-type object 336. The cut-type object 336 may correspond to a particular type of object that can be used to split volumes within the CAD environment 304. For example, when a cut-type object 336 overlaps or shares a volume with some other object in the CAD environment 304, the overlapping volume may be subtracted or removed from the other object. The overlapping volume may be completely removed or may be separated into a separate volume (e.g., thereby creating a third separate volume or object within the CAD environment 304).

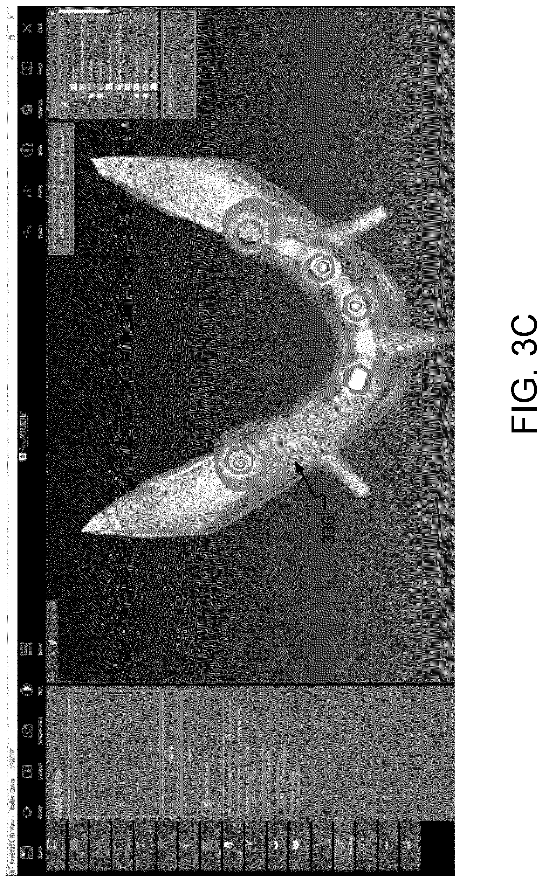

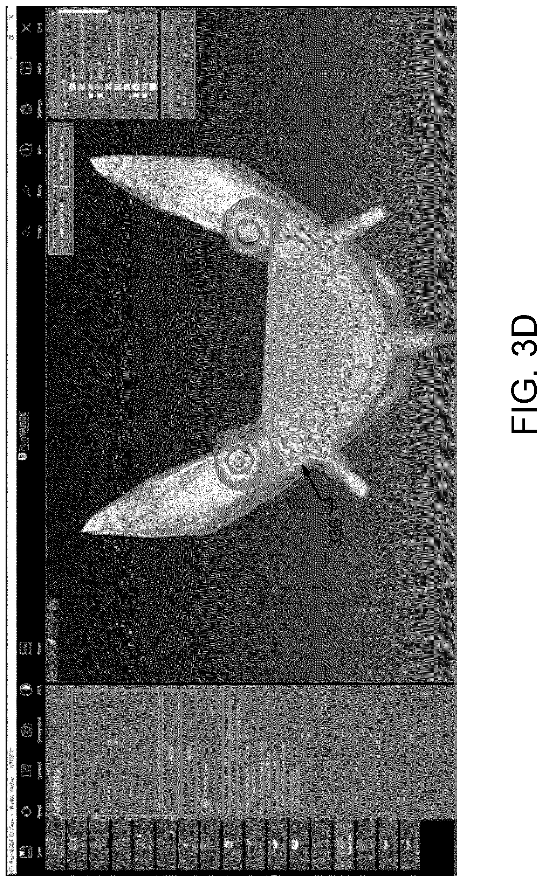

As can be seen in Fig. 3B, a cut-type object 336 can be initially created as a two-dimensional object within a single plane, two planes, or three planes. A cut-type object 336 may correspond to a freeform object that includes one or more control points that are independently capable of manipulation. The control points, as will be described in further detail herein, can be adjusted in one, two, or three dimensions within the CAD environment 304 and the adjustment of control points relative to other control points will result in a different configuration of the cut-type object 336. Furthermore, cut-type objects 336 may be created from scratch or may be created from a template 224. Figs. 3B, 3C, and 3D illustrate a process where a cut-type object 336 is created from scratch and control points are added in a first plane in a sequential fashion. As control points are added, the cut-type object 336 may be expanded within the first plane.

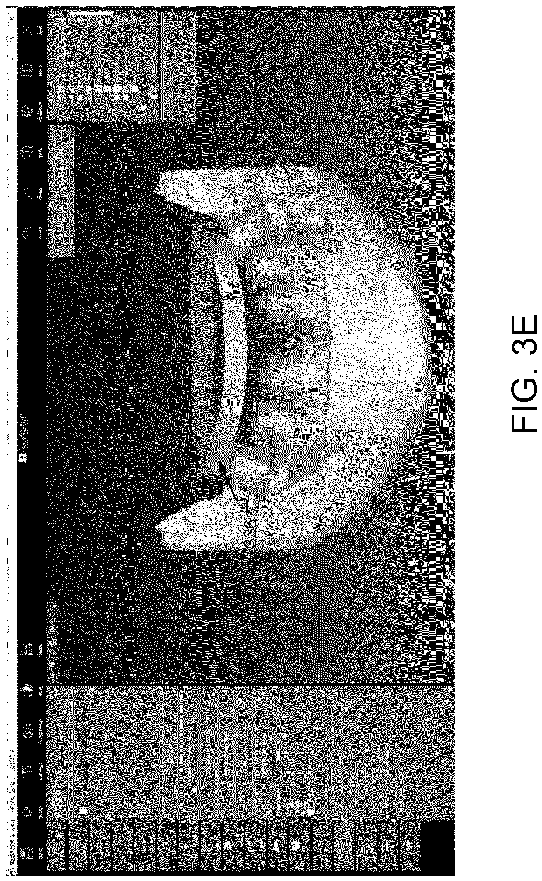

Fig. 3E illustrate that the cut-type object 336 may then be expanded to a volume (e.g., occupying space within three dimensions) using the control points initially defined within the first plane (e.g., the horizontal plane). The cut-type object 336 may alternatively be initially created in three dimensions as a volume, where pairs of control points are automatically created when a user creates a single control point in a single dimension. Said another way, when a control point is created for a new cut-type object 336 in one dimension, the CAD tools 228 may automatically create a second corresponding control point in a second plane such that the corresponding control point exists in the same location of the first control point in two planes, but has a different position in a third plane. In the example of Fig. 3E, control points created in the x-plane and y-plane may result in an automated creation of a corresponding control point in the z-plane to establish the cut-type volume 336.

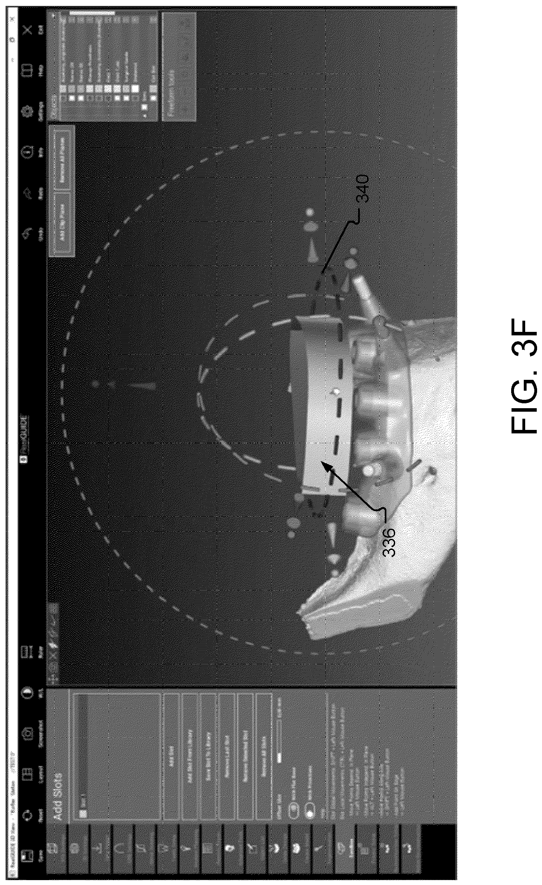

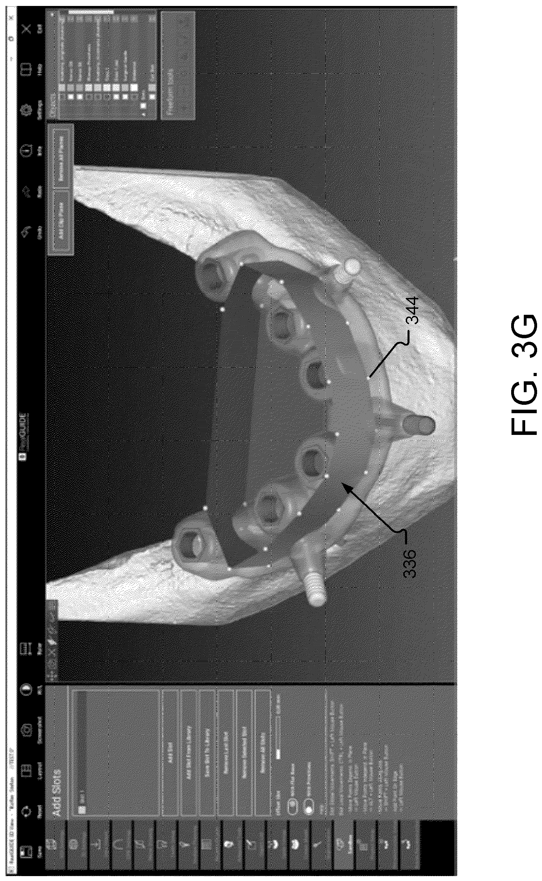

Fig. 3F illustrate rotation handles 340 that may be associated with the cut-type object 336. The rotation handles 340 may be centered about the center of mass of the cut-type object 336 and may facilitate a rotation of the cut-type object 336 in all three dimensions. The rotation handles 340 may allow the cut-type object 336 to be rotated relative to other objects in the CAD environment 304. The rotation handles 340 may also allow a translational movement of the cut-type object 336 within the CAD environment 304. For instance, the rotation handles 340 may be used to rotate the cut-type object 336 in three dimensions and may also be used to translate the cut-type object 336 in three dimensions relative to other objects in the CAD environment 304. As shown in Fig. 3G, the cut-type object 336 can be rotated and translated such that portions of the cut-type object 336 overlap portions of other objects. Fig. 3G also illustrates the independently adjustable control points 344 that may be established on the cut-type object 336 as the cut-type object 336 is being created within the CAD environment 304.

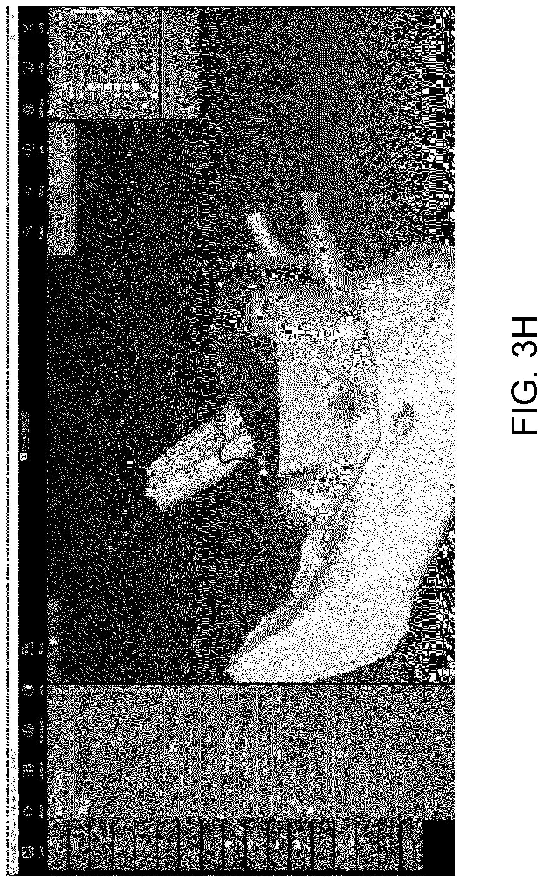

As shown in Figs. 3H and 31, the control points 348 may be independently adjustable relative to other control points 348. This enables the creation and manipulation of the cut-type object 336 as a freeform object and allows the user a unique level of control and customization over the cut-type object 336. In some embodiments, the adjustment of a control point 348 will result in a change of the volume occupied by the cut-type object 336. This may also impact the amount of overlap between the cut-type object 336 and other objects in the CAD environment 304. The control points 348 may be added or deleted to the cut-type object 336 as well. Each control point 348 may provide a mechanism to adjust the size and/or shape of the cut-type object 336 in one, two, or three dimensions.

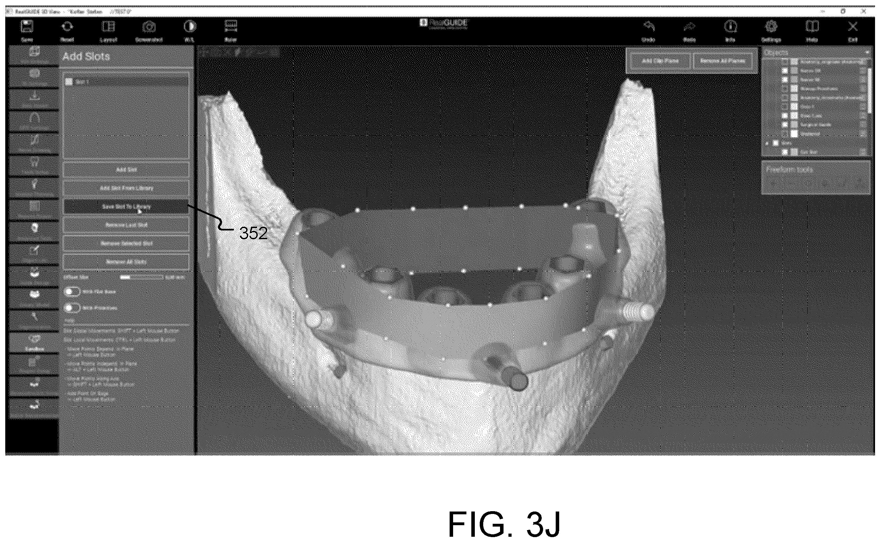

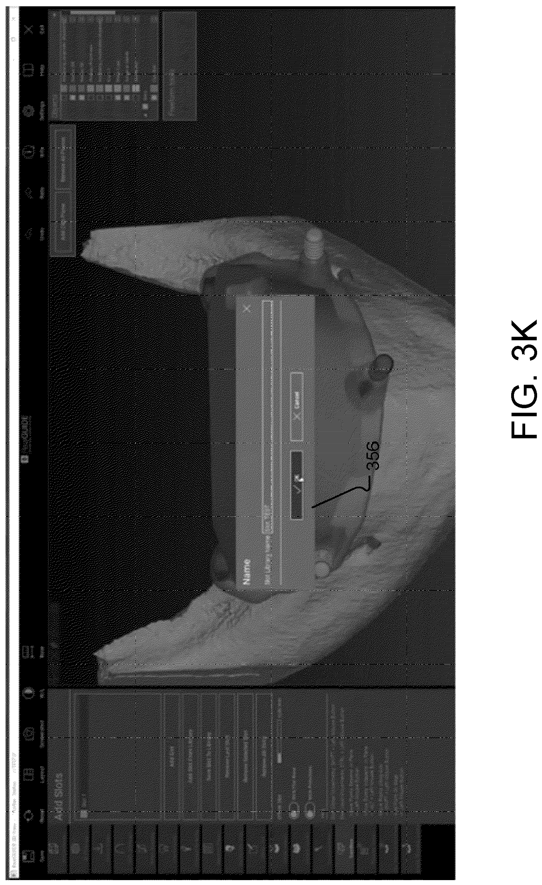

Figs. 3J and 3K show that a cut-type object 336 may be added as a template 244 or may be added to the model library 252. Specifically, the slot creation toolbar 332 may include a save slot icon 352 which allows the user to save a selected object (e.g., cut-type object 336) or combination of objects as a template 244 or into the model library 252. In some embodiments, selection of the save slot icon 352 causes the CAD tools 228 to present a save window 356. Within the save window 356, the user is allowed to name the object(s) currently selected within the CAD environment 304 as a new template 244 or object in the model library 252.

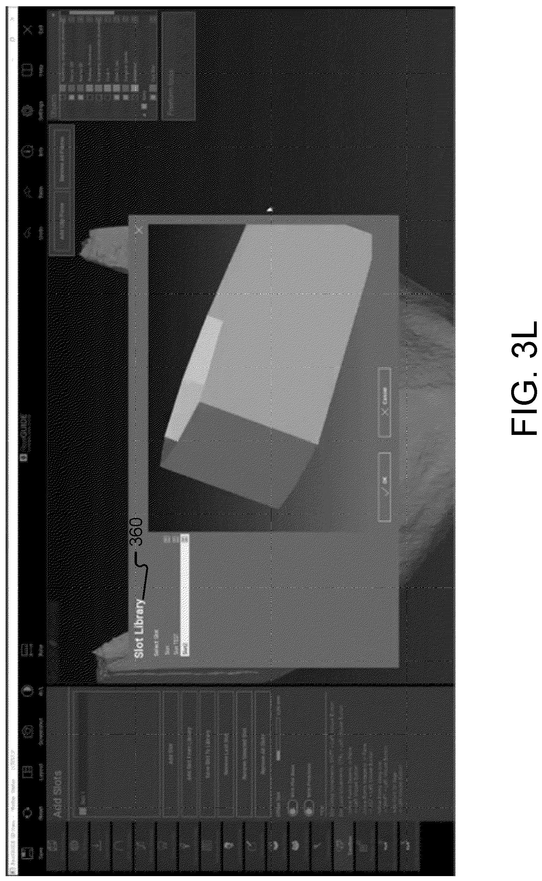

In addition to providing the ability to create new object, the slot creation toolbar 332 may also facilitate the importation of an object into the CAD environment 304 from a template 244 or the model library 252. Specifically, as shown in Fig. 3L, a slot library 360 may be used to view or browse some or all of the objects (e.g., cut-type objects or other objects) that have been saved as a template 244 or object in the model library. An object selected from the slot library 360 may be imported into the CAD environment 304 for manipulation relative to other objects already imported into the CAD environment 304. The object, when initially imported, may have all of the dimensions and control points that existed when the object was created or saved; however, it may be possible to customize the dimensions of the object once imported into the CAD environment. For example, a cut-type object imported from the slot library 360 may have multiple control points 344 that can be adjusted to realize a new freeform object.

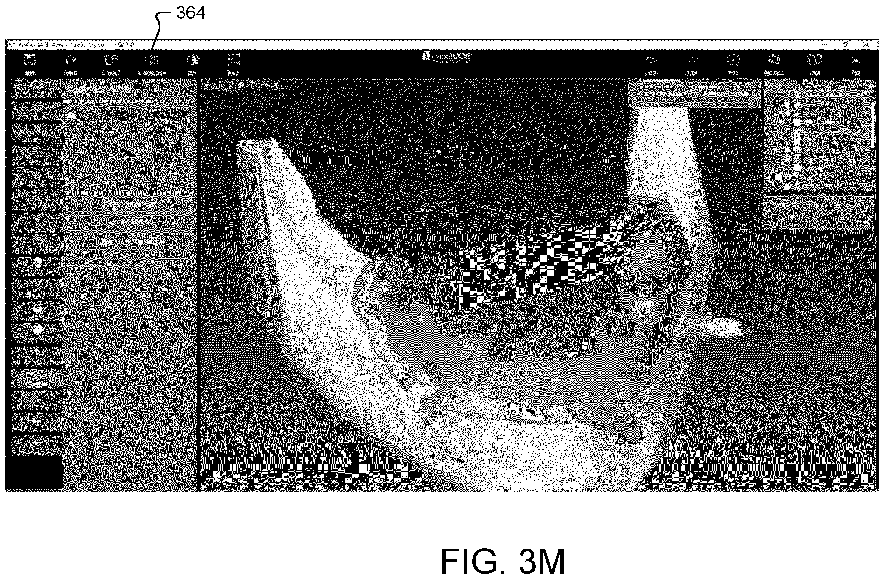

Figs. 3M - 3P illustrate the ability of the cut-type object 336 to create split volumes and/or subtract volumes from existing objects within the CAD environment 304. Specifically, a user (e.g., care provider 108) may be presented with a subtract slot toolbar 364 that helps the user work through the process of creating a split volume and/or subtracting a portion of a volume from another object. In some embodiments, the subtract slot toolbar 364 is only presented or available as an option when a cut-type object 336 is presented in the CAD environment 304 and/or when the cut-type object 336 is at least partially overlapping one or more other objects in the CAD environment 304. When the cut-type object 336 is overlapping at least some of another object, then the overlapping volume may be subtracted from the original volume. This may result in a total removal of the overlapping volume or may result in a split volume that treats the overlapping volume as a new object within the CAD environment 304.

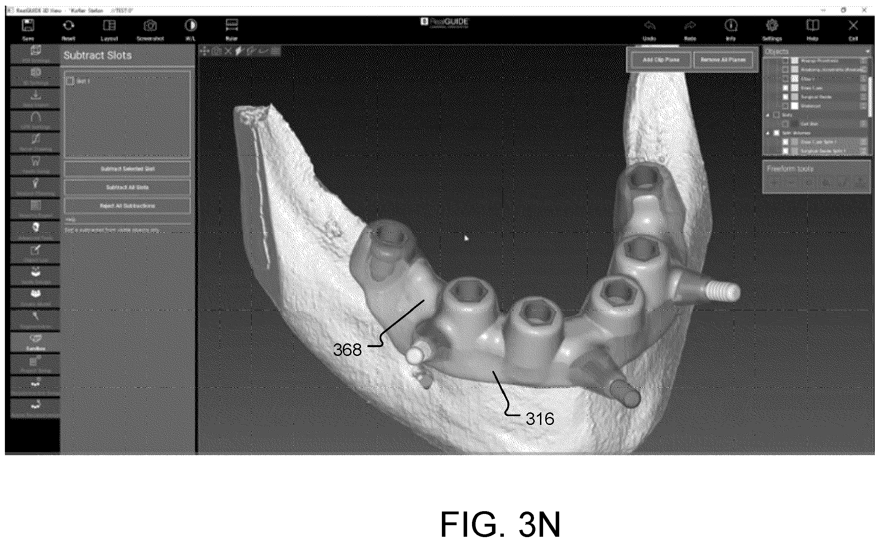

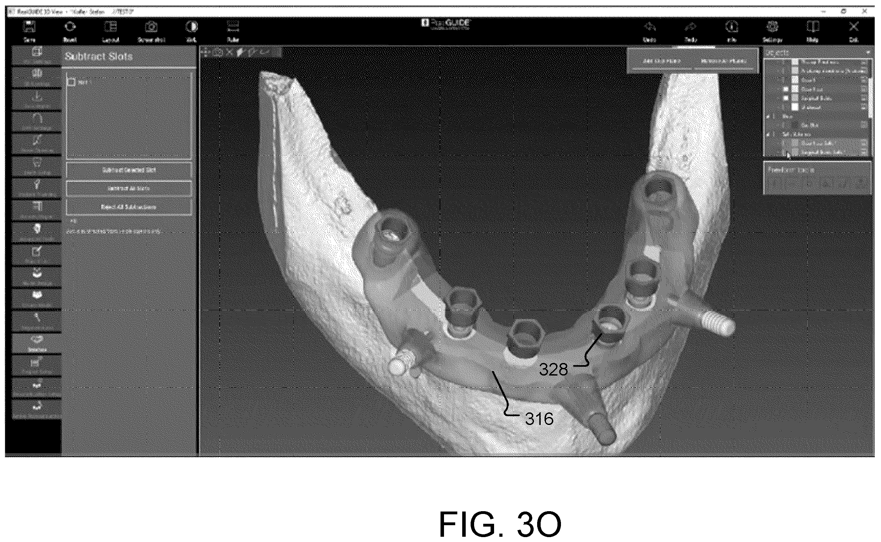

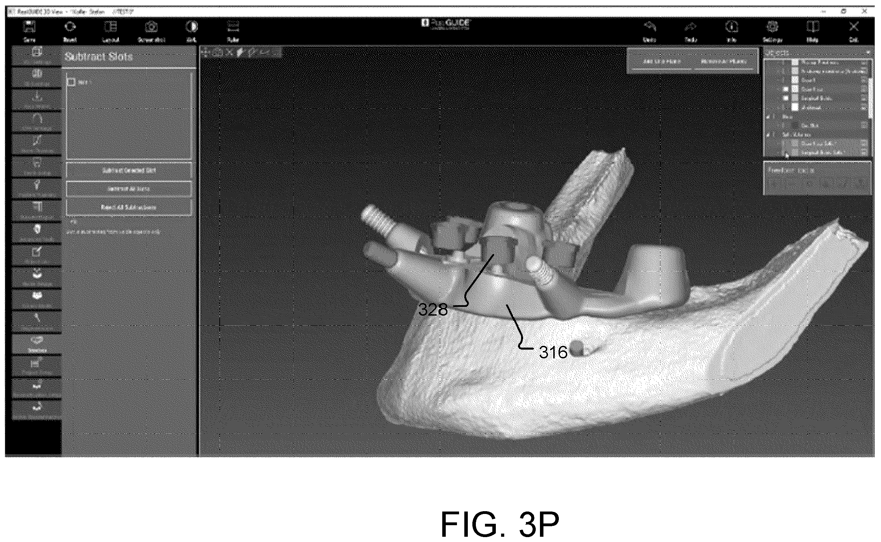

In Fig. 3N, the overlapping volume between object 316 and the cut-type object 336 will become a split volume 368 or new object. When the subtract slot workflow is complete, the portion of the cut-type object 336 that was not overlapping any other object may be removed from the CAD environment whereas the portion of the cut-type object 336 that was overlapping one or more other objects will become a new split volume 368. In some embodiments, the split volume is created using one or more Boolean operations (e.g., AND, SUBTRACT, OR, NOT, XOR, etc.). The Boolean operations may be used to first identify the overlapping volume shared between the cut-type object 336 and other objects 316 in the CAD environment 304. Alternatively or additionally, the Boolean operations may be used to identify the volume(s) of the cut-type object 336 that do not overlap another object 316. The result of performing a subtract slot operation is the creation of a split volume 368, which may still be shown relative to the other object 316. As an example, the split volume 368 may still be considered part of the other object 316, but may also be removable or subtractable relative to the object 316. Figs. 3O and 3P illustrate the object 316 and other objects 328 when the split volume 368 is removed from the CAD environment 304. Removal of the split volume 368 may help to visualize the object 316 and/or to help build stackable guides for a surgical procedure. Because the cut-type object 336 is freeform and capable of customization, the possibility of creating customized stackable guides during a surgical procedure is made relatively easy. Thus, customized surgical guides or stacked surgical guides can be created during a surgical procedure, but in an efficient way, thereby reducing an amount of time that the patient 104 remains on the table or chair 112.

In addition to facilitating the creation of customized object via use of cut-type objects 336, embodiments of the present disclosure also contemplate that freeform objects can be added or merged with other objects in the CAD environment 304. For instance, a workflow is provided in which one or more objects can be merged with other objects to arrive at a resultant object. The object(s) may have one or more control points that are movable in three dimensions. A presentation of the objects in an overlapping arrangement can be shown even while the user manipulates and adjusts a position of control points, thereby changing a size of one or more objects. Eventually, a merge function may be performed using one or more Boolean operations, thereby combining the two separate objects into a resultant object that occupies the volumes and has the shapes of the previously separated objects.

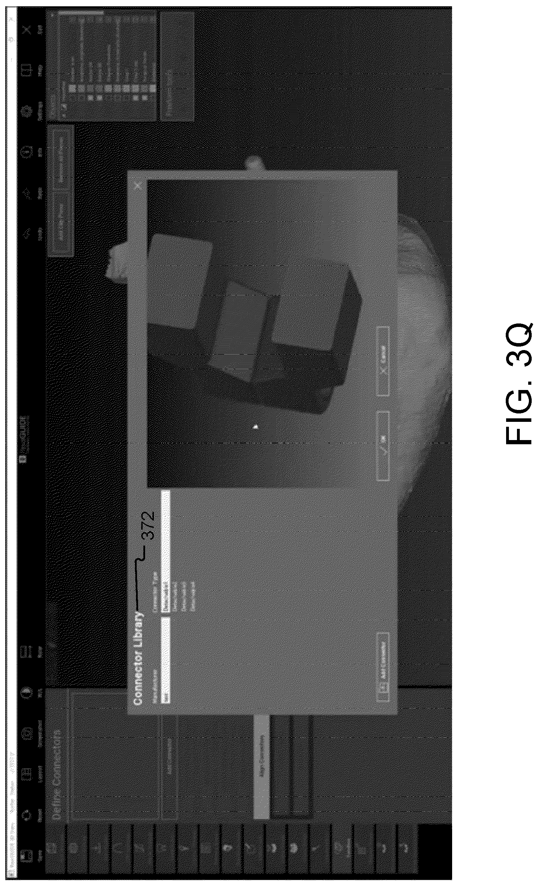

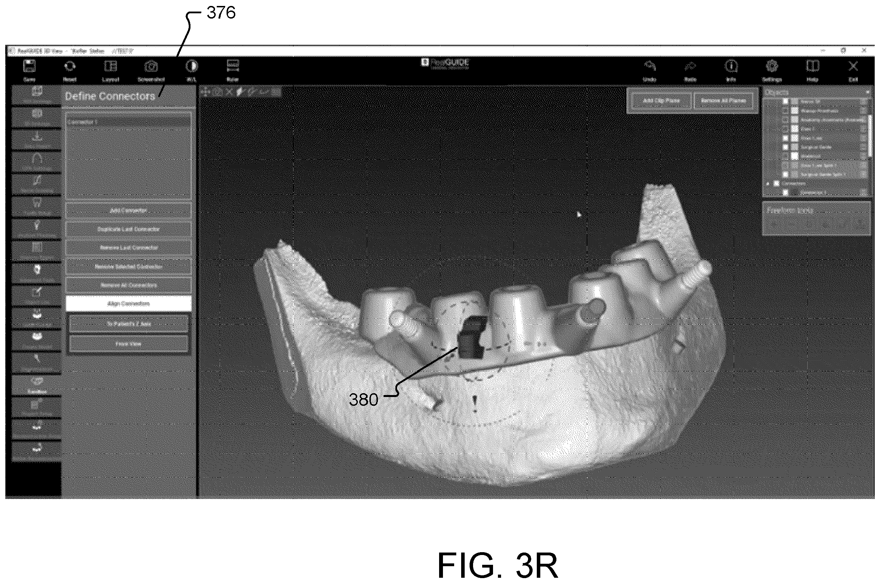

As shown in Fig. 3Q, a connector library 372 may be accessed from a define connectors toolbar. The connector library 372 may show objects that are either templates 244 and/or objects from a model library 252. A selected object may then be imported into the CAD environment 304 as shown in Fig. 3R. Once imported into the CAD environment 304, the object 380 may have rotation handles 340 that enable manipulation or customization of the object within the CAD environment 304. The object 380 may be moved and/or manipulated while still presented as a separate object from the other objects in the CAD environment 304. In some embodiments, the object 380 may be manipulated to overlap at least a portion of another object in the CAD environment 304. As the object 380 is moved within the CAD environment 304 (e.g., rotated, translated, flipped, etc.), the volume occupied by each object in the CAD environment 304 is determined. Furthermore, any volume that is occupied by two or more objects is identified as an overlapping volume in the CAD environment 304.

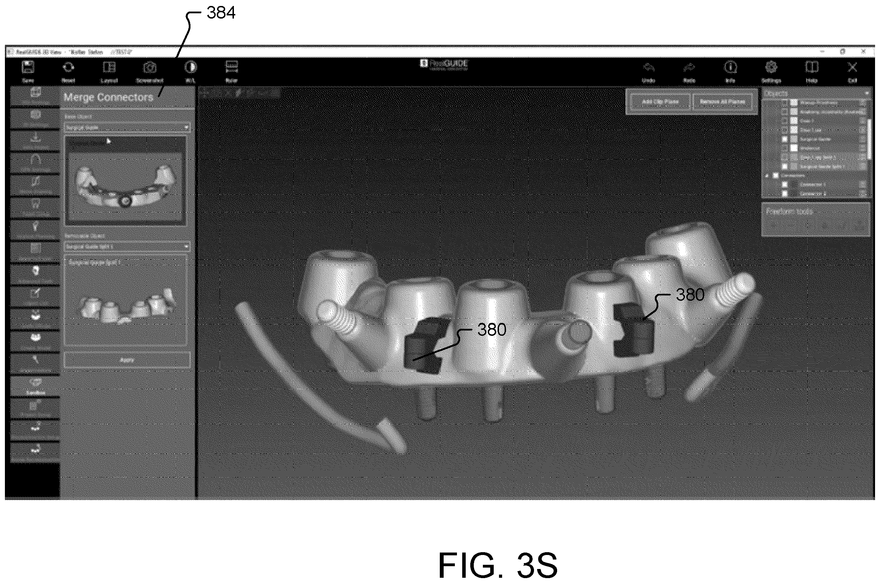

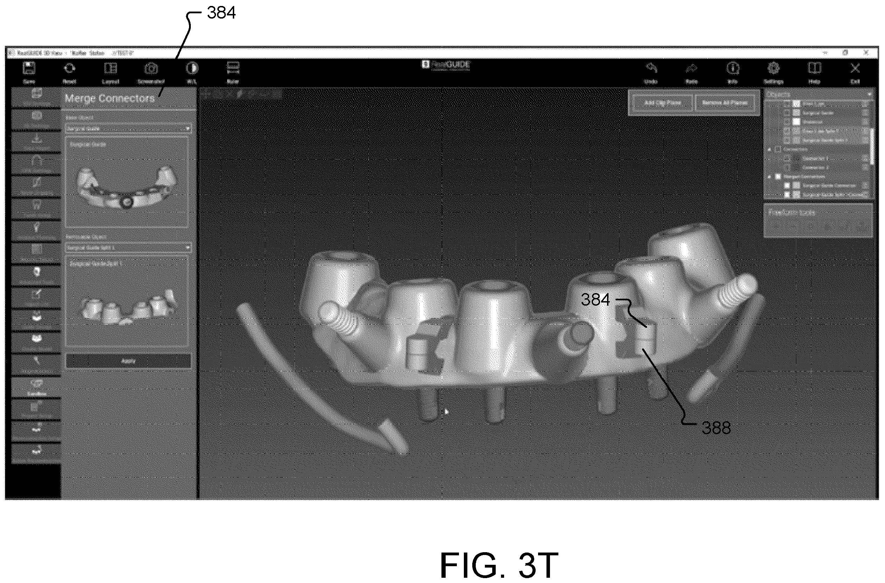

As shown in Fig. 3S, an object 380 may be duplicated or imported into the CAD environment 304 multiple times. Each portion (e.g., volume) of overlap between an object 380 and another object may be determined. The overlapping volume(s) may then be subject to a Boolean operation in which the overlapping volume(s) are subtracted, removed, or otherwise adjusted into a split volume. Alternatively, as shown in Fig. 3S, a merge connector toolbar 384 may be provided that facilitates the merging or combining of objects 380 with other objects in the CAD environment 304. In some embodiments, objects 380 may only be merged with another object of a particular type. For example, but without limitation, objects 380 may only be eligible for merging with other non-anatomical objects. Thus, when a merge function is selected from within the merge connector toolbar 384, only the objects eligible for merger with the object 380 may remain displayed while other objects not eligible for merger with the object 380 are removed from view. When the user selects a final merge option from the merge connector toolbar 384, a Boolean operation (e.g., AND) may be used to join or merge the objects 380 with other objects based on their overlapping volumes. Fig. 3T illustrates resultant objects 388, 392 after the merger of a connector-type object 380 with other objects in the CAD environment 304. In some embodiments, the resultant objects 388, 392 may correspond to two pieces of a stackable surgical guide. The upper object 392 may correspond to a first portion of a split volume previously created using a cut-type object, which was subsequently merged with the connector-type object 380. Because the objects 388, 392 were initially created using a volume-splitting function (e.g., via use of the cut-type object 336), the interface between object 388 and 392 is perfectly aligned. Physical objects manufactured according to the dimensions of objects 388, 392 in the CAD environment 304 will mate perfectly (e.g., within machining tolerances) to one another.

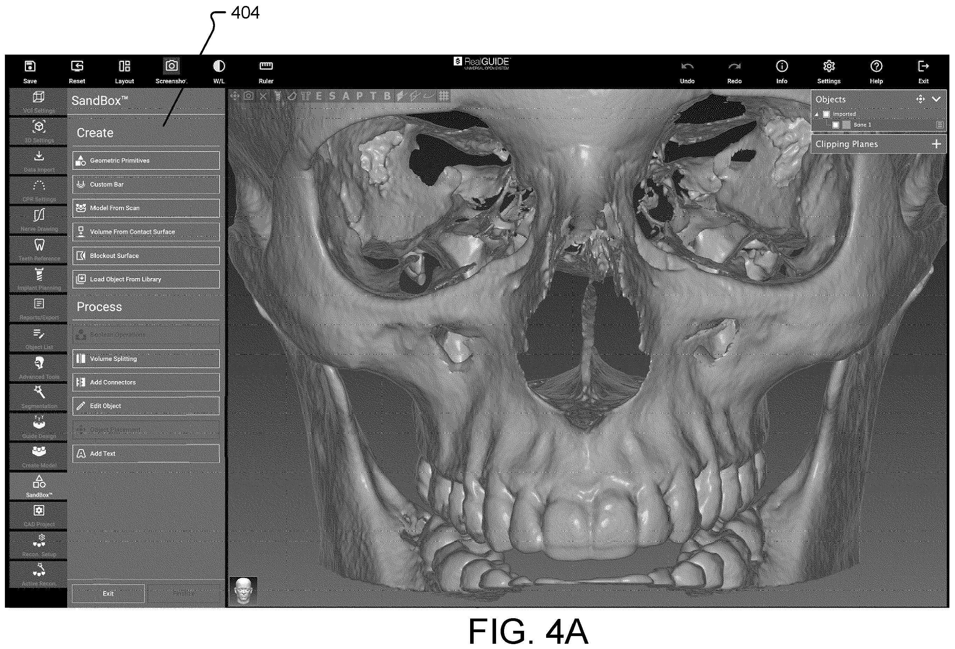

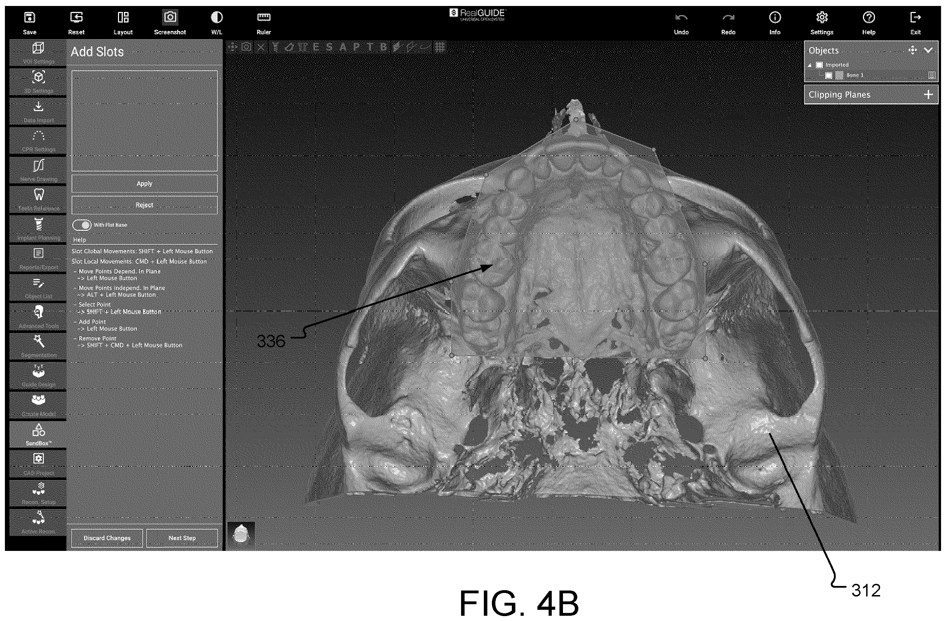

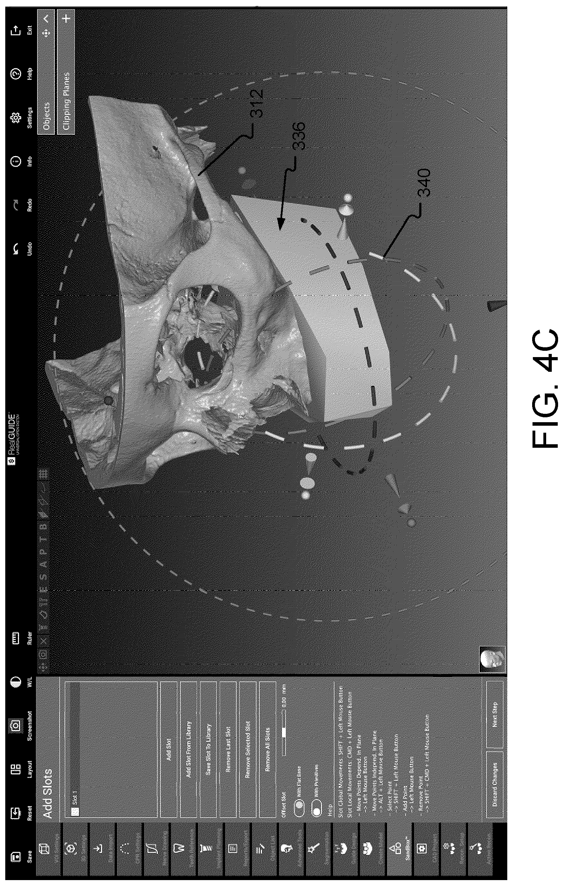

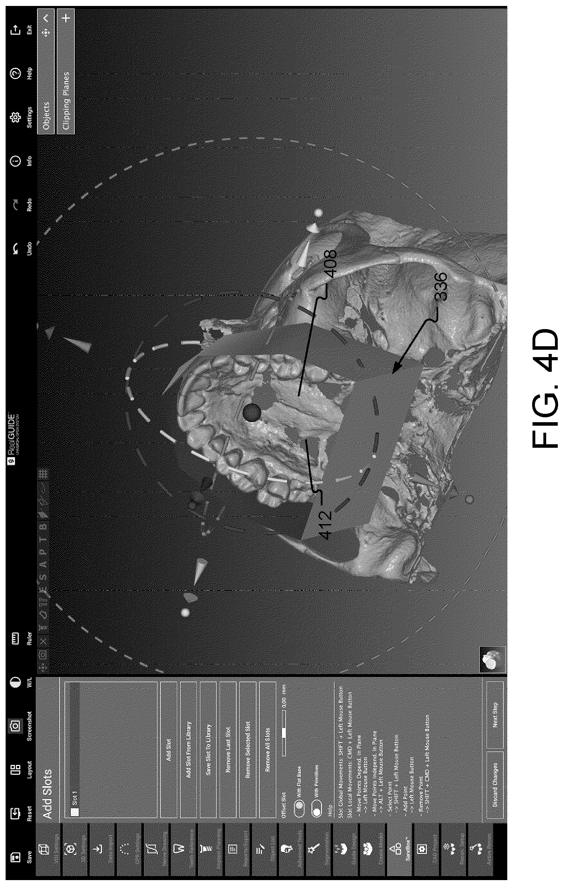

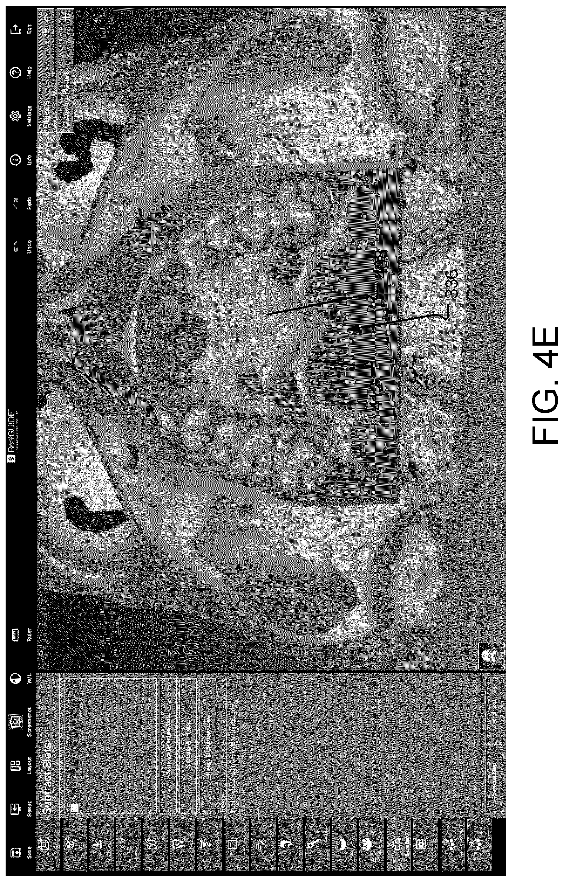

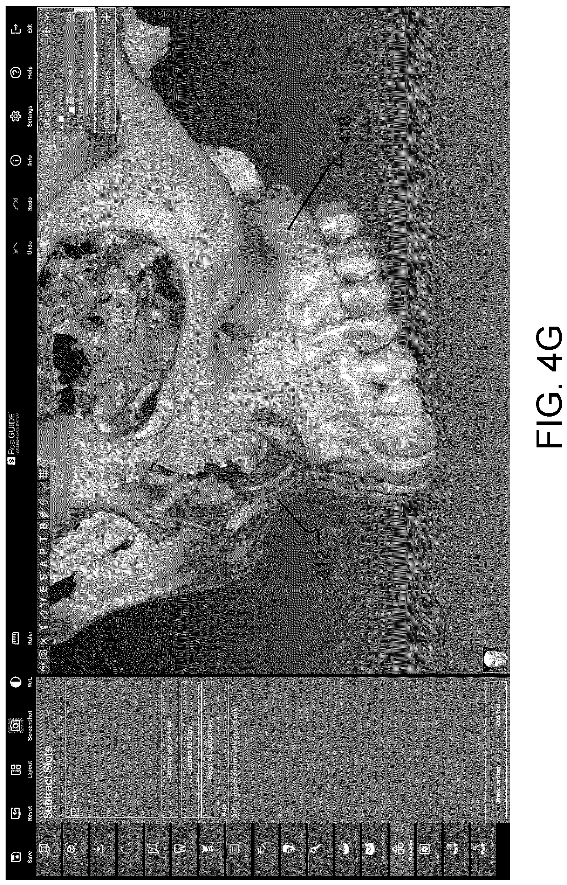

With reference now to Figs. 4A - 4J, another workflow or set of workflows that are made possible by the CAD tools 228 will be described in accordance with at least some embodiments of the present disclosure. The CAD tools 228 may facilitate the planning of surgical cuts, bone grafts, and/or creation of surgical guides that help define a planned cut. Fig. 4A illustrates a sandbox toolbar 404 that facilitates the planning of surgical procedures or manoeuvres for a surgical procedure. In some embodiments, the sandbox toolbar 404 may provide access to the slot creation toolbar 332. As discussed above, a user may be allowed to create a new cut-type object 336 from scratch as a freeform object. The cut-type object 336 may initially be created within the CAD environment 304 without overlapping any other object. However, as the user manipulates the cut-type object 336, the size, orientation, and/or position of the cut-type object 336 may change to eventually overlap at least a part of another object 312. In some embodiments, the object 312 being overlapped with the cut- type object 336 may correspond to an anatomical-type object. Once the cut-type object 336 is established within the CAD environment 304, the cut-type object 336 may be manipulated using a rotation handle 340.

As the user manipulates the cut-type object 336, an overlapping volume 408 may be established between the cut-type object 336 and the other object 312. The size and shape of the overlapping volume 408 may change as the cut-type object 336 is manipulated relative to the other object 312. There may also be a volume boundary 412 that is determined at the intersection of the objects 312, 316. In some embodiments, the volume boundary 412 may correspond to a plane or collection of planes that are not included in the overlapping volume 408, but that will be exposed if/when the overlapping volume 408 is removed from the object 312. As such, the cut-type object 336 and visualization of the volume boundary 412 may be helpful in planning a surgical procedure, such as a maxillary resection, where the bone from the overlapping volume 408 is cut and then moved to better fit the patient's occlusion and aesthetics.

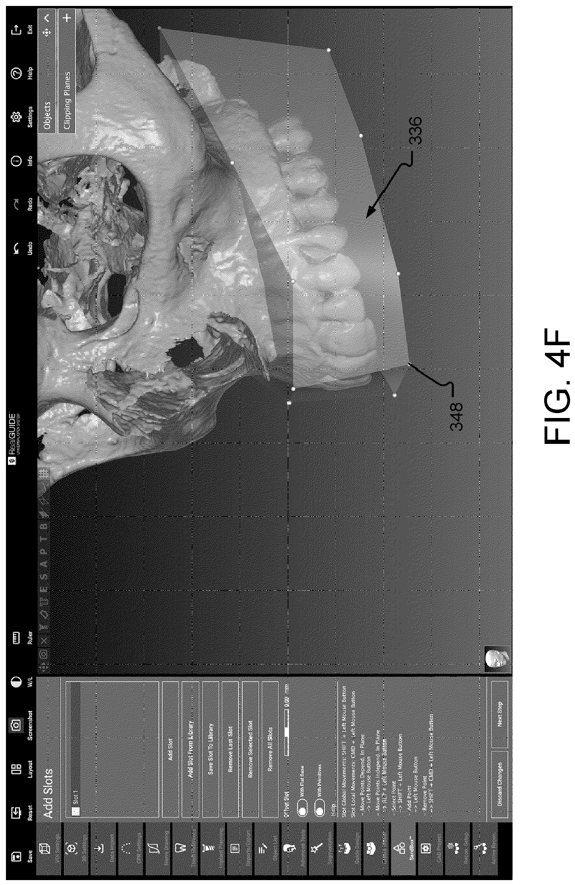

As can be seen in Fig. 4F, the cut-type object 336 may include one or more control points 348 that allow the manipulation of the cut-type object 336 in one, two, or three dimensions. The cut- type object 336 may be manipulated via the control points 348 even when shown as overlapping with other objects 312 and the overlapping volume 408 may be recalculated each time the cut-type object 336 is manipulated.

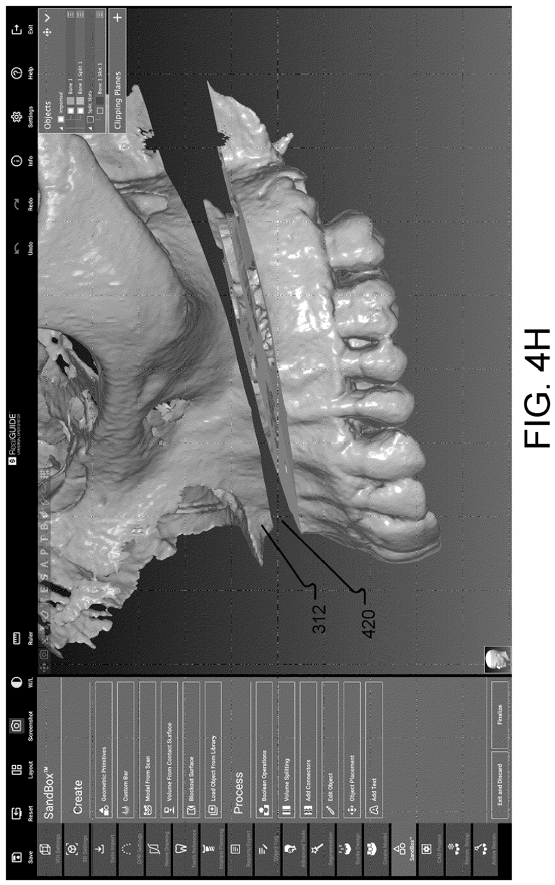

The overlapping volume 408 may eventually be subtracted from the object 312 to create a split volume 416. The split volume 416 may correspond to a portion of the object 312 that was overlapping with the cut-type object 336 when the subtract function was selected. The split volume 416 may then be treated as a separate object from the object 312 within the CAD environment 304. As shown in Fig. 4H, the split volume 416 may be separated from the object 312 to reveal a cut portion or void 420 that results if the split volume 416 is physically removed from the object 312. The dimensions of the void 420 can be useful to visualize and plan a cut of the object 312.

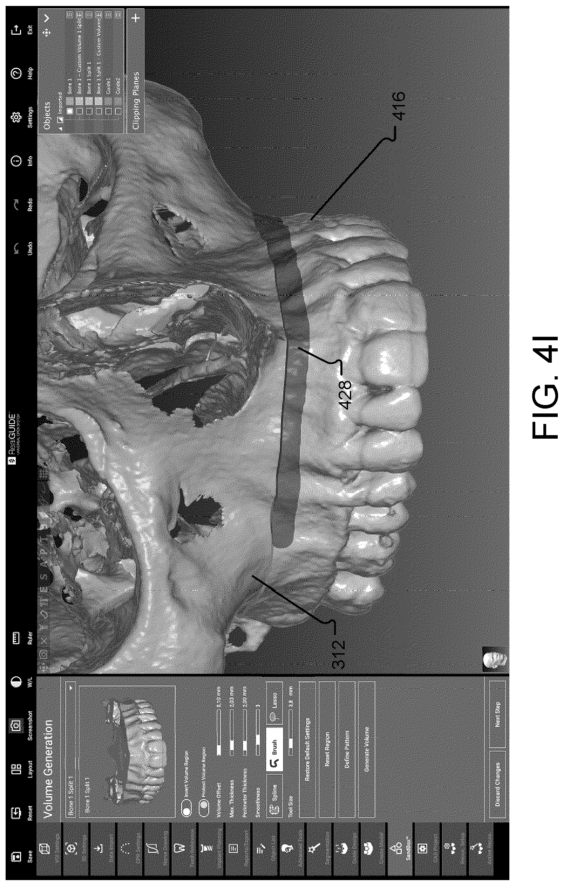

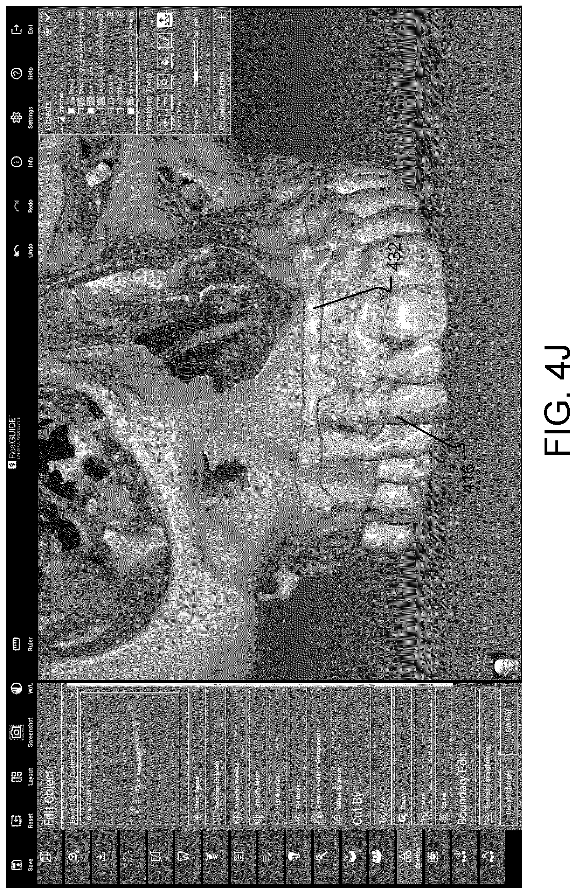

As shown in Figs. 4I and 4J, a volume generation toolbar may provide access to a border tool that allows the user to define an additional border 428 around the split volume 416. When the additional border is drawn around the surface of object 312 and encloses the split volume 416, the additional border 428 may be used to automatically generate a perimeter of a surgical guide 432. The surgical guide may have a predefined thickness that is generated in response to completing the additional border 428. The surgical guide 432 may initially generated automatically in response to the additional border 428 being completed around the split volume 416 when the split volume 416 is established in an anatomical object 312. The thickness of the surgical guide 432 may initially be generated with substantial conformity to the surface of the object 312. The width of the surgical guide 432 may correspond to a distance between the outer border of the split volume 416 and the additional border 428. As with other objects in the CAD environment 304, the surgical guide 432 may initially be created with a plurality of control point by selecting the contact surface 428 on the anatomy surface. The contact surface 428 may also be removed from or added to the surgical guide 432 to allow the user to customize the size and dimensions of the surgical guide 432 within the CAD environment.

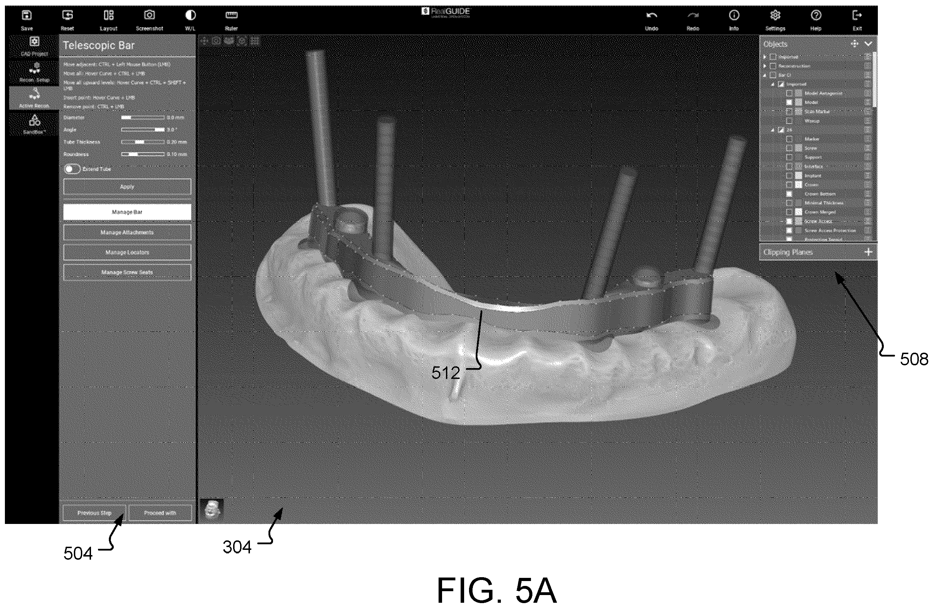

Referring now to Figs. 5A - 5E, workflows for creating other types of reconstructions in addition to surgical guides or stacked surgical guides will be described in accordance with at least some embodiments of the present disclosure. In Fig. 5A, the GUI 300 presents a prosthetic reconstruction wizard side panel 504 along with an image of a 3D model or object in the CAD environment 304. An object control toolbar 508 may be presented alongside the CAD environment 304 and may enable further manipulation or adjustment of objects presented in the CAD environment 304. This reconstruction is intended as an example of the application of this concept to any suitable type of prosthetic reconstruction utilizing CAD software.

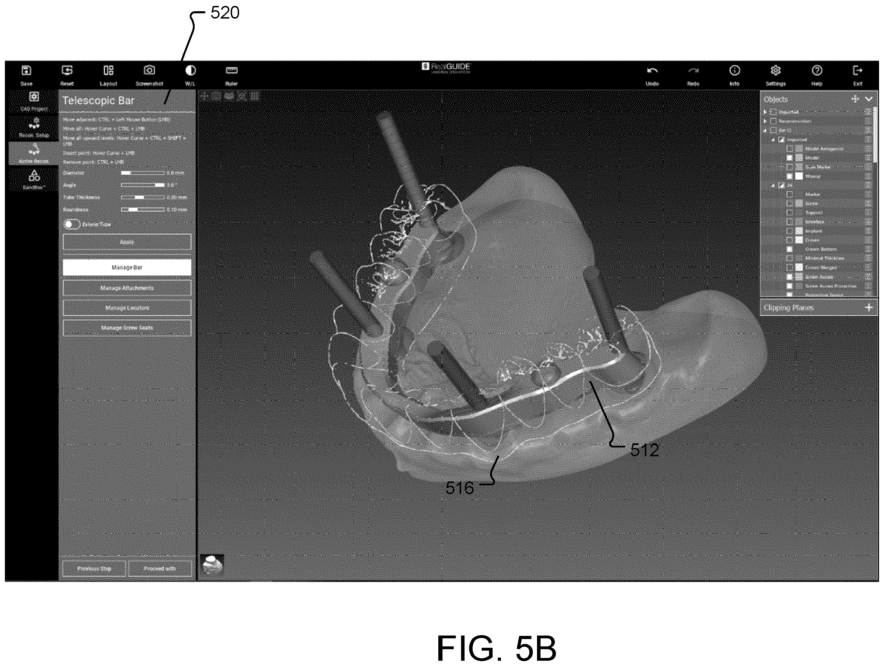

Fig. 5B illustrates a telescopic bar reconstruction 516 anchored to the patient bone by implants. The illustrated bar structure 512 provides load distribution on extended parts of the prosthetic reconstruction making the overall reconstruction 516 robust. Since sometime, despite the customization of their shape, dimensions of the bar structure 512 could interfere with their anatomical coupled overstructure, thereby making it desirable or necessary to split part of the structure 512.

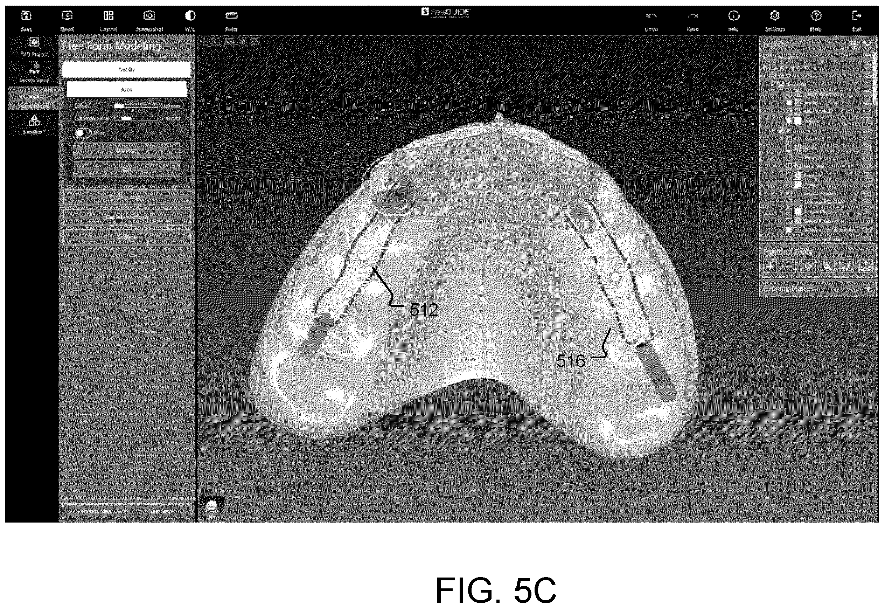

As shown in Fig. 5C, the placement of the control points that define the splitting area follows many of the similar concepts depicted and described herein. The same is replicated in the rest of the 3D control points functions as described herein.

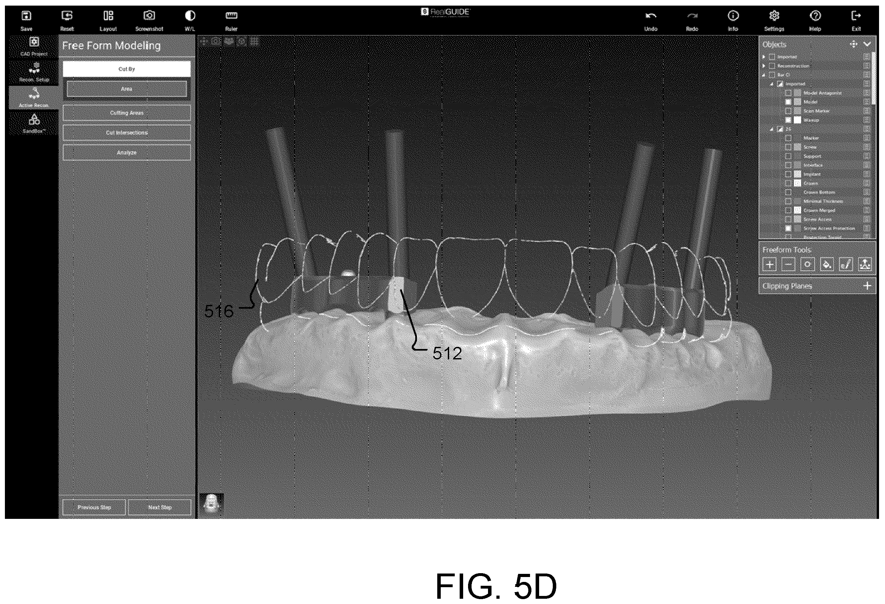

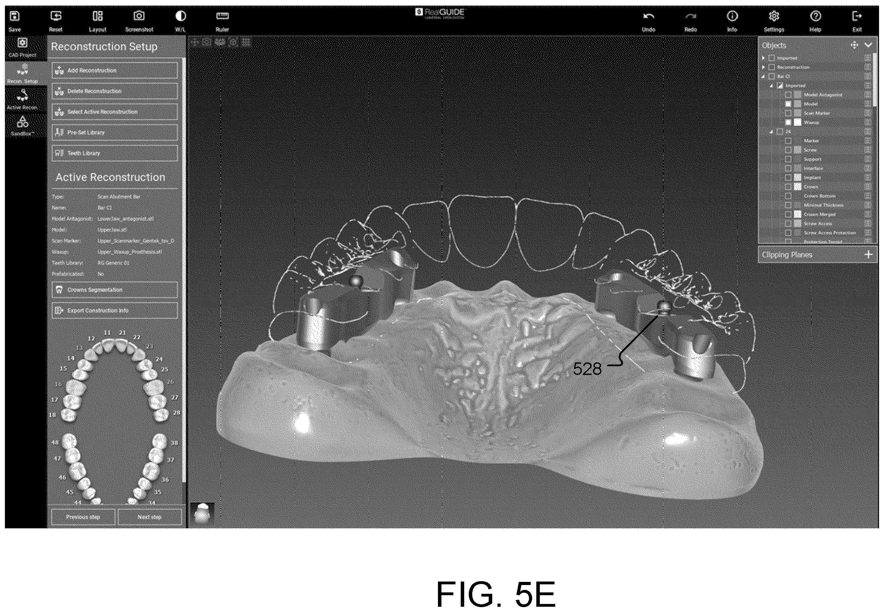

Figs. 5D and 5E illustrate the result of the splitting defined in Fig. 5C being applied to the bar structure 512. As described herein, an object, such as the bar structure 512, may be split into one or more resultant portions 528. The resultant portions may have the same shape as the bar structure 512 minus the volume of the cut-type object. This subtraction or splitting operation can be performed in the surgical planning, in the prosthetic CAD, and/or in the SandBox environments.

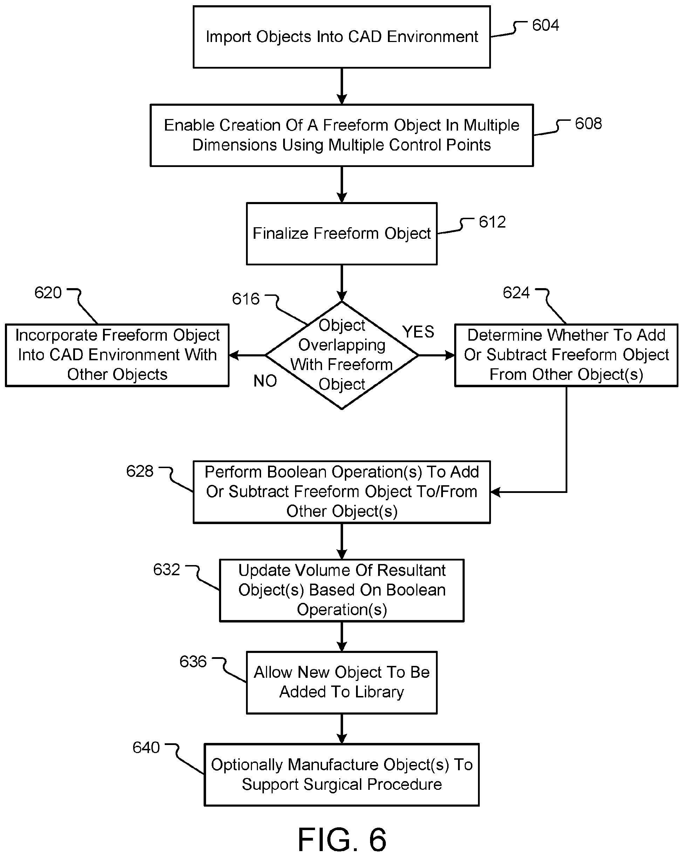

With reference now to Figs. 6-8, various methods and will be described in accordance with at least some embodiments of the present disclosure. The methods described herein may correspond to methods that can be performed in connection with any of the workflows previously described herein and may be performed by one or more components of a system 100 or computing device 200. Moreover, the order of steps illustrated in any particular method should not be construed as limiting. Rather, steps of any method may be performed in any order and/or may be combined with steps of other methods without departing from the scope of the present disclosure.

Referring initially to Fig. 6, a first method is illustrated in accordance with at least some embodiments of the present disclosure. The method begins by importing one or more objects into the CAD environment 304 (step 604). The method continues by enabling the creation of a freeform object, where dimensions and/or surfaces of the freeform object are capable of being changing in multiple dimensions (step 608). The freeform object may correspond to any type of object depicted and/or described herein. The freeform object may have one or more control points that enable manipulation of the freeform object or surfaces thereof in one, two, or three dimensions. Each of the control points of the freeform object may be configured for independent manipulation.

The method continues when the freeform object has been finalized (step 612). Once finalized, the determination is made as to whether or not the freeform object overlaps at least one other object in the CAD environment 304 (step 616). In some embodiments, the query of step 616 may be answered affirmatively if at least some volume in the CAD environment 304 is occupied by both the freeform object and at least one other object. If the query of step 616 is answered negatively, then the method continues by incorporating the freeform object into the CAD environment 304 without adjusting a dimension or size of any other object already existing in the CAD environment 304.

On the other hand, if the query in step 616 is answered affirmatively, then the method continues by determining whether the freeform object will be added or subtracted from other object(s) (e.g., objects that are overlapping with the freeform object) (step 624). A Boolean operation of combination of Boolean operations may then be performed to add/merge or subtract/remove the freeform object from any other object(s) overlapped with the freeform object (step 628). In some embodiments, the Boolean operation(s) may be performed to create a split volume across a portion of overlap between the freeform object and another object. Said another way, the Boolean operation(s) may be performed and the resultant volumes may be created based on overlaps between two or more objects in the CAD environment 304 (step 632).

The method may then continue by allowing the user to add the resultant object to a collection of templates and/or a model library (step 636). The method may also continue with the CAD tools 228 providing one or more electronic files to a manufacturing system 140 to enable the manufacturing system 140 to produce a physical component according to dimensions of the object in the electronic file received from the CAD tools 228 (step 640).

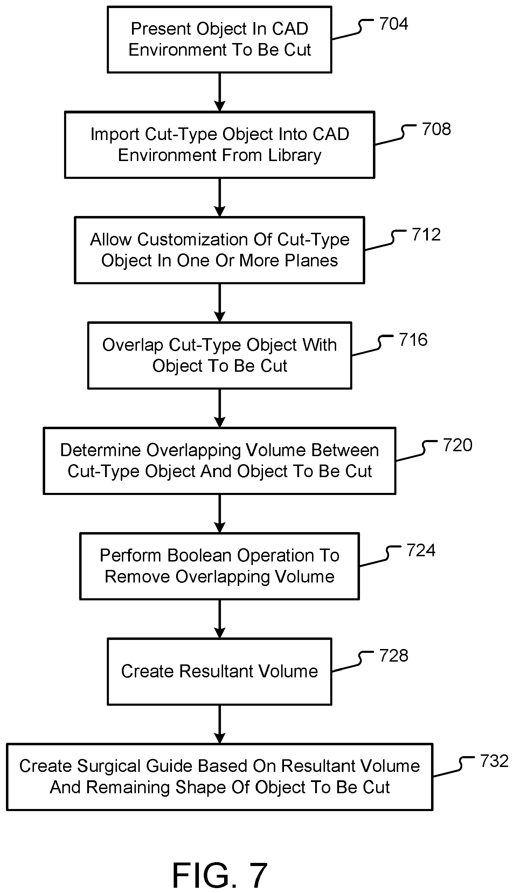

With reference now to Fig. 7, another method will be described in accordance with at least some embodiments of the present disclosure. The method may include one or more steps used to create a surgical guide or collection of surgical guides (e.g., stacked surgical guides). The method begins with the presentation of one or more objects in a CAD environment 304 (step 704). The initial object(s) presented within the CAD environment 304 may include anatomical objects, non-anatomical objects, freeform objects, cut-type objects, connector objects, and the like.

The method may continue when a cut-type object is imported into the CAD environment (step 708). In some embodiments, the cut-type object may correspond to a freeform object that is created from scratch (e.g., from two, three, four, or more user defined control points). Alternatively, the cut-type object may be imported into the CAD environment from a library of templates.

The method then continues by allowing the user of the CAD tools 228 to manipulate and customize the cut-type object in one or more planes (step 712). In some embodiments, the cut-type object may include a number of control points, each of which are independently capable of being moved in one, two, or three planes relative to other control points of the cut-type object. The independent control over different control points may allow for the customization of the freeform object, regardless of whether the object was imported as a template or is being created from scratch.

The method may then continue by determining an amount of overlap between the cut-type object and another object presented in the CAD environment 304 (step 716). Specifically, but without limitation, the overlapping volume occupied by the cut-type object and another object may be correspond to a volume of the other object that is to be cut or removed during a surgical procedure (step 720).

Once the position of the cut-type object is finalized, the method may continue by determining the overlapping volume and performing one or more Boolean operations to remove the overlapping volume or define the overlapping volume as a split volume of the object being overlapped by the cut-type object (step 724). A resultant volume or combination of volumes (e.g., multiple split volumes) may be created based on the performance of the Boolean operations (step 728). The method may then continue with the CAD tools 288 communicating with the manufacturing system 140 to create one or more surgical guides based on the resultant volume (e.g., the split volume) and the remaining shape of the object to be cut (step 732). As can be appreciated, this step may include the creation of one, two, three, or more surgical guides to support any number of surgical procedures or maneuvers during a surgical procedure.

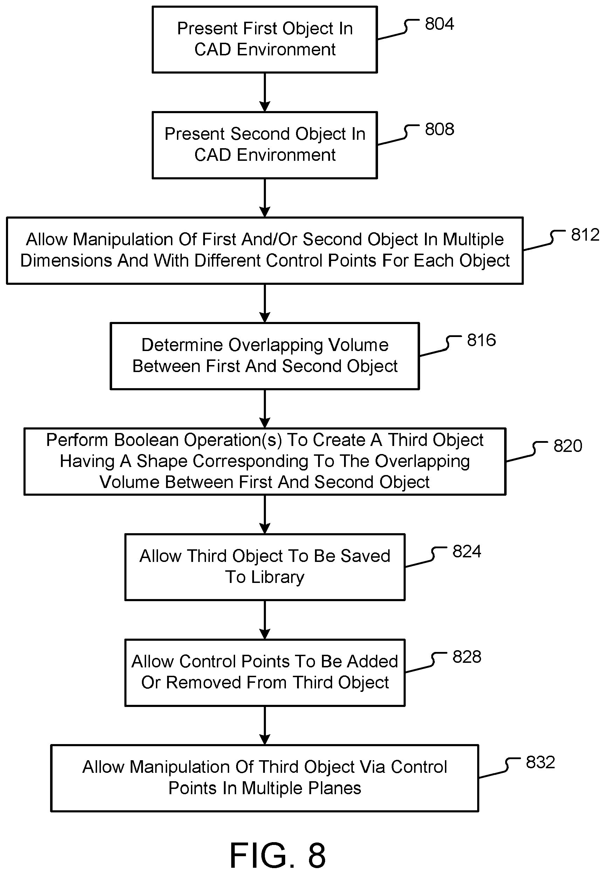

Referring now to Fig. 8, another method will be described in accordance with at least some embodiments of the present disclosure. The method may include one or more steps for creating, adjusting, combining, and subtracting objects in a CAD environment 304. In some embodiments, the method may be initiated by presenting a first object in a CAD environment 304 (step 804). The first object may correspond to a freeform object, a cut-type object, a connector object, a template-based object, an object imported from a model library, an anatomical object, a non-anatomical object, or the like. The method may also include presenting a second object in the CAD environment 304 (step 808). The second object may be similar to or different from the first object.

The method may also include allowing a user of the CAD tools 228 to manipulate the first object and/or second object within the CAD environment 304. In this step, the user may be allowed to manipulate or customize one or both of the first object and second object. The object(s) may be manipulated in one or more dimensions using one or many different control points (step 812). As object(s) are manipulated, the CAD tools 228 may calculate and recalculate the volumes in the CAD environment that have overlap between the first object and the second object (step 816).

Once the user indicates that the manipulation of the object(s) has been completed and a next operation is desired (e.g., a split operation, a cut operation, a merge operation, etc.), the method may continue with the CAD tools 228 performing one or more Boolean operations to create a third object having a shape corresponding to the overlapping volume between the first and second object (step 820). In some embodiments, the overlapping volume may become a third object, which may also be referred to as a split volume of the first and/or second object.

The third object (e.g., the overlapping volume) may correspond to a complex object having many different planes and multiple control points. The method may continue by allowing the third object to be saved into a library or be saved as a template for later use (step 824). The method may also include allowing the user to add or remove control points to/from the third object (step 828). The user may then manipulate and/or change a shape of the third volume by moving the control point(s) of the third volume (step 832). After adjustment, the third shape may then be resaved as another template or may replace the existing template created in step 824.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

## Claims

1. A computing device, comprising:
a processor; and
memory including data that, when processed by the processor, enables the computing device to:
import a freeform object into a Computer-Aided Drafting (CAD) environment;
facilitate a customization of the freeform object within the CAD environment by independent manipulation of one or more control points in at least two dimensions, wherein the one or more control points are defined at a surface of the freeform object and, when manipulated, result in a changed configuration of the freeform object in the at least two dimensions;
determine an overlapping volume in the CAD environment that is shared between the freeform object and another object; and
perform one or more Boolean operations to convert the overlapping volume into a split volume.

2. The computing device of claim 1, wherein the split volume is created as a new portion of the object that is overlapped with the freeform object.

3. The computing device of claim 1, wherein the freeform object comprises a three-dimensional volume and wherein the at least two dimensions comprises at least three dimensions.

4. The computing device of claim 1, wherein the Boolean operation is used to subtract the freeform object from the object and wherein the split volume becomes a third object within the CAD environment.

5. The computing device of claim 1, wherein the Boolean operation comprises at least one of AND, SUBTRACT, OR, NOT, and XOR.

6. The computing device of claim 1, further comprising a communication interface that facilitates machine-to-machine communication between the processor and a manufacturing system.

7. The computing device of claim 6, wherein an electronic file describing at least a portion of the split volume is provided to the manufacturing system to enable production of a surgical guide.

8. The computing device of claim 6, wherein the data defines dimensions of the split volume and wherein the split volume shares at least some dimensions with the object.

9. The computing device of claim 6, wherein the manufacturing system is configured to produce a physical object according to the data during a surgical procedure where a patient is sedated and positioned in at least one of a chair and table.

10. The computing device of claim 1, wherein the data, when processed by the processor, further enables the computing device to:
merge the freeform object with the object.

11. The computing device of claim 1, wherein the object corresponds to an anatomical object.

12. The computing device of claim 11, wherein the freeform object corresponds to a cut-type object and wherein the overlapping volume corresponds to a portion of the anatomical object to be cut during a surgical procedure.

13. The computing device of claim 12, wherein the data, when processed by the processor, further enables the computing device to:
enclose the overlapping volume with a border; and
automatically generate an object representing a surgical guide within the border, wherein the object representing the surgical guide comprises a void that is substantially aligned with the overlapping volume.

14. The computing device of claim 1, wherein the data, when processed by the processor, further enables the computing device to:
save an object template into a template library, wherein the object template comprises dimensions that correspond to dimensions of the freeform object after manipulation of the one or more control points.

15. The computing device of claim 1, wherein the one or more control points of the freeform object are capable of manipulation even when the freeform object overlaps the object in the CAD environment.

16. The computing device of claim 15, wherein the overlapping volume is recalculated after every instance of the one or more control points being moved.

17. The computing device of claim 1, wherein the data, when processed by the processor, further enables the computing device to:
duplicate the split volume within the CAD environment for purposes of planning a bone surgery with the split volume.

18. A surgical system, comprising:
a computing device configured to import an object into a Computer-Aided Drafting (CAD) environment, enable creation of one or more control points on a surface of the object, and further enable customization of the object within the CAD environment via independent manipulation of the one or more control points in two or more dimensions even when the object overlaps at least one other object in the CAD environment.

19. The surgical system of claim 18, wherein the object corresponds to a non-anatomical element and wherein the at least one other object corresponds to an anatomical object created with one or more images of a patient anatomy, the surgical system further comprising:
a manufacturing system that interfaces with the computing device and is configured to produce a physical object having dimensions based on dimensions of an overlapping volume shared between the object and the at least one other object within the CAD environment.

20. A computer-implemented method, comprising:
importing a first object into a Computer-Aided Drafting (CAD) environment;
importing a second object into the CAD environment;
enabling independent manipulation of the first object and/or second object relative to one another within the CAD environment even when the first object overlaps the second object;
determining an overlapping volume in the CAD environment that is shared between the first object and the second object;
creating a third volume based on performing one or more Boolean operations to convert the overlapping volume into the third volume; and
presenting the third volume within the CAD environment as a portion of the first object and/or second object.
